# EUROPEAN PATENT APPLICATION

(11) **EP 4 834 913 A1**
(43) Date of publication of application: **04.11.2026**
(21) Application number: 24911548.6
(22) Date of filing: 27.12.2024
(51) Int. Cl.: C07H 21/02, A61K 31/713, A61P 1/16

(54) **TARGETING COMPOUND FOR USE IN DELIVERY AND USE THEREOF**

(30) Priority: 27.12.2023 CN 202311834901; 30.05.2024 CN 202410695515; 06.09.2024 CN 202411253447
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); AN, Ke, Wuhan, Hubei 430075 (CN); ZHAO, Xin, Wuhan, Hubei 430075 (CN); JIANG, Wen, Wuhan, Hubei 430075 (CN); WU, Qian, Wuhan, Hubei 430075 (CN); LI, Cong, Wuhan, Hubei 430075 (CN); KE, Chaoqi, Wuhan, Hubei 430075 (CN); JIA, Yimin, Wuhan, Hubei 430075 (CN)
(74) Representative: M. Zardi & Co S.A.
(86) International application number: PCT/CN2024/143392
(87) International publication number: WO 2025/140632

(57) **Abstract**

Provided in the present disclosure is a compound represent by formula (I), or a stereoisomer, tautomer or pharmaceutically acceptable salt thereof:

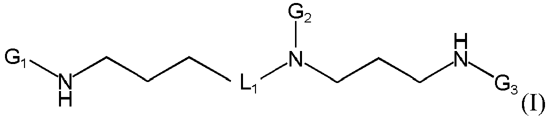

## Description

### PRIORITY INFORMATION

This application claims priority to and benefits of Chinese patent application No. 202311834901.6, filed with China National Intellectual Property Administration on December 27, 2023, Chinese patent application No. 202410695515.1, filed with China National Intellectual Property Administration on May 30, 2024, and Chinese patent application No. 202411253447.X, filed with China National Intellectual Property Administration on September 06, 2024, the entire contents of which are incorporated herein by reference.

### FIELD

The present disclosure relates to the technical field of biopharmaceuticals, and more particularly, to a targeting compound for delivery and use thereof.

### BACKGROUND

Nucleic acid pharmaceutical agents (e.g., siRNA, ASO, etc.) are playing an increasingly important role in gene therapy. They specifically target pathogenic genes or proteins through binding or cleavage mechanisms, thus inhibiting or promoting the expression of certain genes/proteins. Ligand compounds represent one of the core key technologies in the development of nucleic acid pharmaceutical agents. By conjugating with nucleic acids, such ligand compounds can carry the nucleic acids and deliver them in a targeted manner to target cells. Therefore, there is an urgent need to develop a novel targeting compound.

### SUMMARY

The present disclosure aims to solve, at least in part, the technical problems existing in the related art. To this end, the present disclosure provides a targeting compound for delivering an active pharmaceutical agent, which is capable of carrying the active pharmaceutical agent and delivering the active pharmaceutical agent to target cells. In particular, the present disclosure provides a targeting compound for delivering nucleic acids, which is capable of carrying an oligonucleotide (e.g., siRNA) and delivering the oligonucleotide to target cells.

In a first aspect of the present disclosure, the present disclosure provides a compound represented by formula (I), or a stereoisomer, tautomer or pharmaceutically acceptable salt thereof: 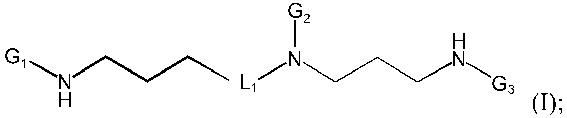
where: L₁ is -N(R₁)-C(O)-(C₁₋₁₀ alkylene)- or -N(G₄)-L₂-;
L₂ is C₁₋₆ alkylene substituted with R₁;
G₁, G₂, G₃, and G₄ are each independently 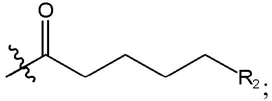
R₂ is a residue of a substituted or unsubstituted saccharide;
R₁ is -(C₀₋₁₀ alkylene)-C(O)-R₁₀; and
R₁₀ is a leaving group.

According to an embodiment of the present disclosure, L₂ is C₂₋₄ alkylene substituted with R₁.

According to an embodiment of the present disclosure, L₂ is -CH₂-CH(R₁)-CH₂-.

According to an embodiment of the present disclosure, L₁ is -N(R₁)-C(O)-(C₁₋₆ alkylene)-.

According to an embodiment of the present disclosure, L₁ is -N(R₁)-C(O)-(C₁₋₆ alkylene)- and R₁ is -(C₁₋₆ alkylene)-C(O)-R₁₀.

According to an embodiment of the present disclosure, L₁ is -N(G₄)-CH₂-CH(R₁)-CH₂-.

According to an embodiment of the present disclosure, L₁ is -N(G₄)-CH₂-C(R₁)H-CH₂- and R₁ is -C(O)-R₁₀.

According to an embodiment of the present disclosure, R₁ is -(C₀₋₆ alkylene)-C(O)-R₁₀.

According to an embodiment of the present disclosure, R₁₀ is selected from the group consisting of

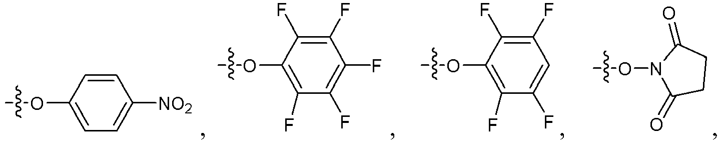

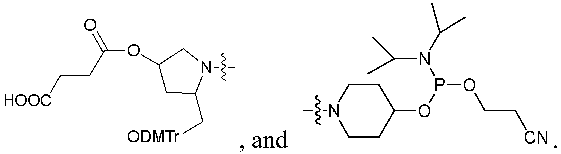

According to an embodiment of the present disclosure, 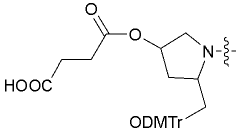 is

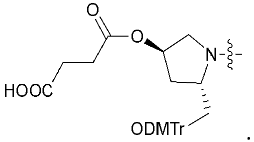

According to an embodiment of the present disclosure, R₁ is selected from the group consisting of

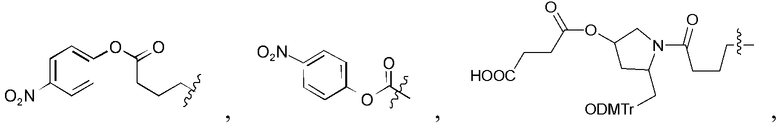

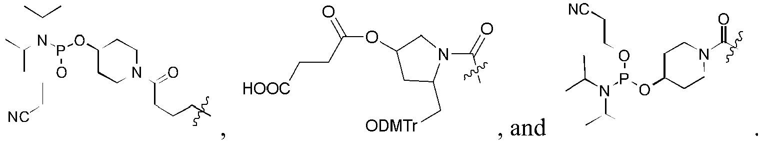

According to an embodiment of the present disclosure, 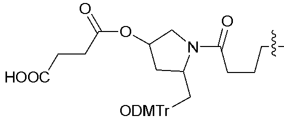 is

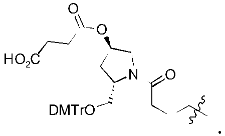

According to an embodiment of the present disclosure, 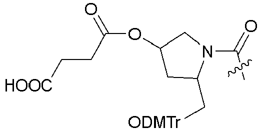 is

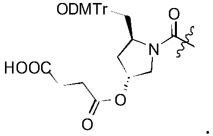

According to an embodiment of the present disclosure, the saccharide is glucose, lactose, galactose, or fructose.

According to an embodiment of the present disclosure, R₂ in G₁, G₂, G₃, and G₄ is each independently a residue of a compound selected from the group consisting of: N-acetylgalactosamine (GalNac), N-trifluoroacetylgalactosamine, N-formylgalactosamine, N-propionylgalactosamine, N-n-butyrylgalactosamine, and N-isobutyrylgalactosamine.

According to an embodiment of the present disclosure, R₂ in G₁, G₂, G₃, and G₄ is identical.

According to an embodiment of the present disclosure, G₁, G₂, G₃, and G₄ are each independently

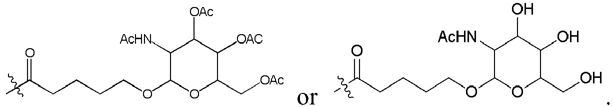

According to an embodiment of the present disclosure, G₁, G₂, G₃, and G₄ are each independently

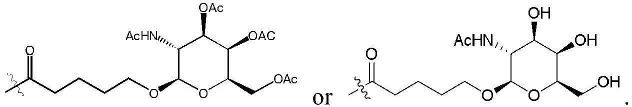

According to an embodiment of the present disclosure, the compound represented by formula (I) has a structure represented by formula (Ia): 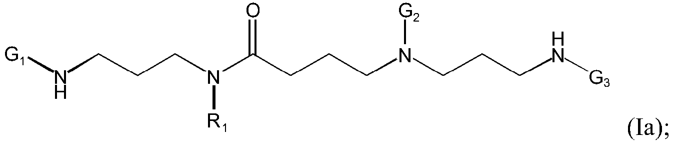
where: R₁ is -(C₁₋₆ alkylene)-C(O)-R₁₀; and
G₁, G₂, G₃, and G₄ are as defined above.

According to an embodiment of the present disclosure, in formula (Ia), R₁ is selected from the group consisting of 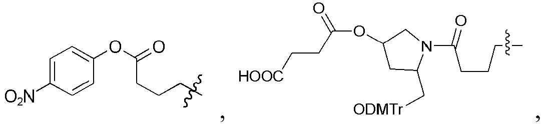 and 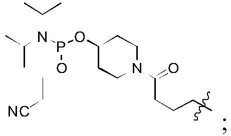 G₁, G₂, G₃, and G₄ are each independently 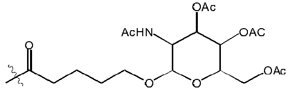 or

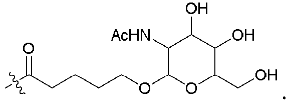

According to an embodiment of the present disclosure, in formula (Ia), R₁ is selected from the group consisting of 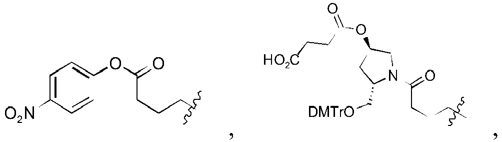 and 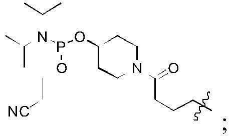 G₁, G₂, G₃, and G₄ are each independently 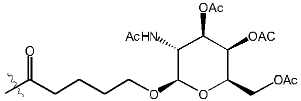 or

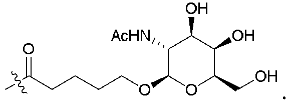

According to an embodiment of the present disclosure, the compound represented by formula (I) has a structure represented by formula (Ib): 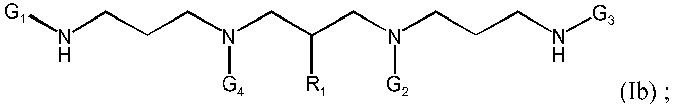
where: R₁ is -C(O)-R₁₀; and
G₁, G₂, G₃, and G₄ are as defined above.

According to an embodiment of the present disclosure, in formula (Ib), R₁ is selected from the group consisting of 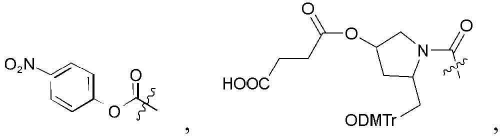 and 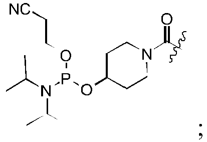 G₁, G₂, G₃, and G₄ are each independently 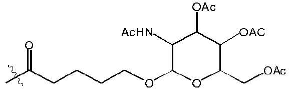 or

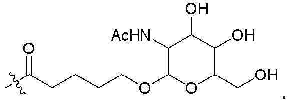

According to an embodiment of the present disclosure, in formula (Ib), R₁ is selected from the group consisting of 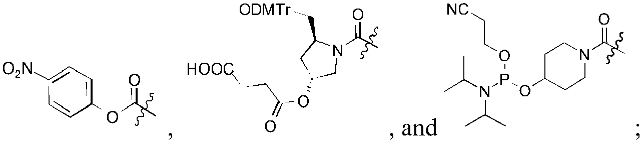 G₁, G₂, G₃, and G₄ are each independently 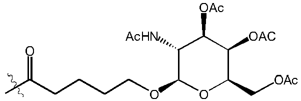 or

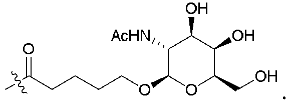

According to an embodiment of the present disclosure, the compound represented by formula (I) has any one of the following structures:

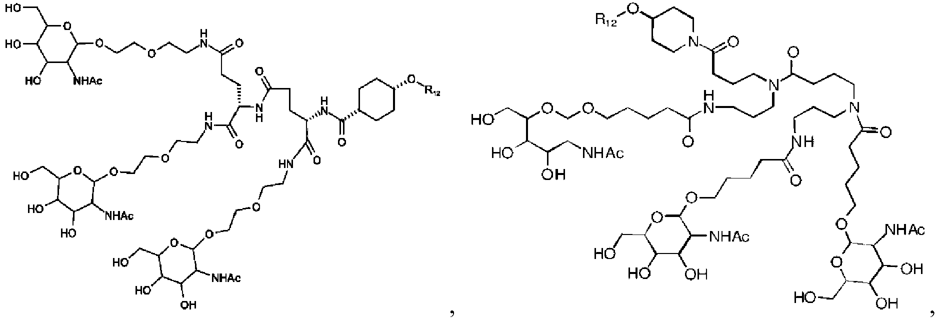

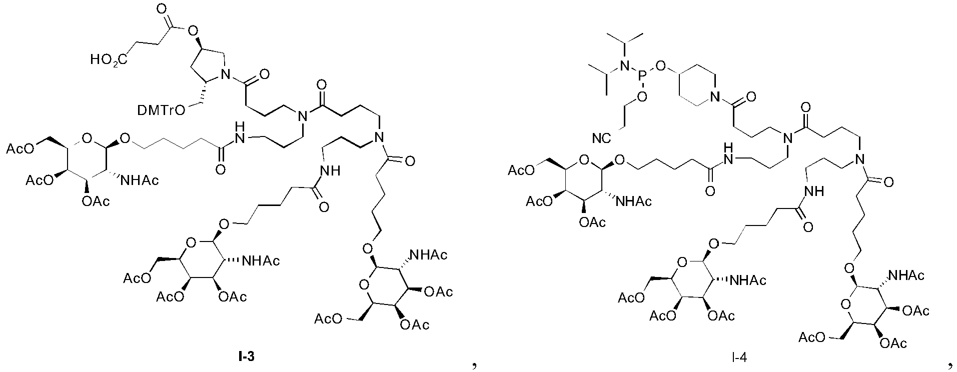

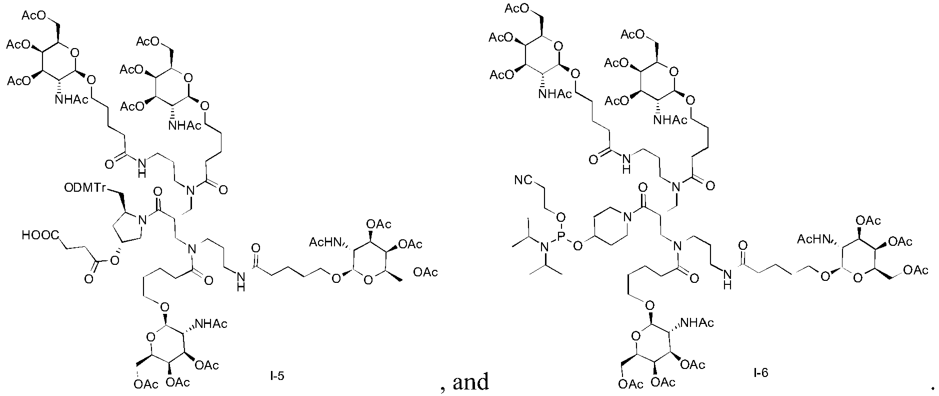

In a second aspect of the present disclosure, the present disclosure provides a compound, or a stereoisomer, tautomer or pharmaceutically acceptable salt thereof, having a structure represented by formula (II): 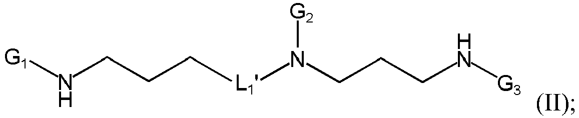
where: L₁' is -N(R₁')-C(O)-(C₁₋₁₀ alkylene)- or -N(G₄)-L₂'-;
L₂' is C₁₋₆ alkylene substituted with R₁';
G₁, G₂, G₃, and G₄ are each independently 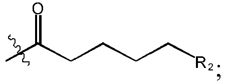
R₂ is a residue of a substituted or unsubstituted saccharide;
R₁' is 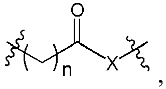 where n is any integer ranging from 0 to 10; and
X is absent or a linker;
the linker, if present, is -NH-(C₁₋₁₀ alkylene)-O-, -NH-(C₁₋₁₀ alkylene)-S-, -C₄₋₁₀ heterocycloalkylene-O-, -C₄₋₁₀ heterocycloalkylene-S-, -C₄₋₁₀ heterocycloalkylene-(C₁₋₆ alkylene)-O- optionally substituted with one or more Rₐ groups, or -C₄₋₁₀ heterocycloalkylene-(C₁₋₆ alkylene)-S- optionally substituted with one or more Rₐ groups, where the one or more Rₐ groups is each independently selected from -OH and -C₁₋₆ alkylene.

According to an embodiment of the present disclosure, X is a linker.

In the present disclosure, the structure represented by formula (II) can serve as a ligand targeting liver cells, and particularly, can promote the targeting and delivery of a nucleic acid pharmaceutical agent, enabling nucleic acids to be delivered to desired cells and/or tissues in vivo (e.g., targeted delivery of an oligonucleotide for targeted silencing of a target gene in target cells and/or tissues), thus effectively preventing and/or treating related diseases.

According to an embodiment of the present disclosure, L₂' is C₂₋₄ alkylene substituted with R₁'.

According to an embodiment of the present disclosure, L₂' is -CH₂-CH(R₁')-CH₂-.

According to an embodiment of the present disclosure, n is any integer ranging from 0 to 6.

According to an embodiment of the present disclosure, L₁' is -N(R₁')-C(O)-(C₁₋₆ alkylene)- or -N(G₄)-CH₂-CH(R₁')-CH₂-.

According to an embodiment of the present disclosure, L₁' is -N(R₁')-C(O)-(C₁₋₆ alkylene)-.

According to an embodiment of the present disclosure, L₁' is -N(R₁')-C(O)-(C₁₋₆ alkylene)-, where n is any integer ranging from 1 to 6.

According to an embodiment of the present disclosure, L₁' is -N(G₄)-CH₂-CH(R₁')-CH₂-.

According to an embodiment of the present disclosure, L₁' is -N(G₄)-CH₂-CH(R₁')-CH₂-, where n is 0.

According to an embodiment of the present disclosure, the linker is -NH-(C₁₋₁₀ alkylene)-O-, -NH-(C₁₋₁₀ alkylene)-S-, -C₄₋₁₀ heterocycloalkylene-O-, -C₄₋₁₀ heterocycloalkylene-S-, -C₄₋₁₀ heterocycloalkylene-(C₁₋₆ alkylene)-O- optionally substituted with one or more -OH, or -C₄₋₁₀ heterocycloalkylene-(C₁₋₆ alkylene)-S- optionally substituted with one or more -OH.

According to an embodiment of the present disclosure, the linker is -NH-(C₄₋₈ alkylene)-O-, -NH-(C₄₋₈ alkylene)-S-, -C₄₋₁₀ heterocycloalkylene-O-, -C₄₋₁₀ heterocycloalkylene-S-, -C₄₋₁₀ heterocycloalkylene-(C₁₋₃ alkylene)-O- substituted with one or more -OH, or -C₄₋₁₀ heterocycloalkylene-(C₁₋₃ alkylene)-S- substituted with one or more -OH.

According to an embodiment of the present disclosure, the linker is selected from the group consisting of -NH-(C₁₋₁₀ alkylene)-O-, -NH-(C₁₋₁₀ alkylene)-S-, -C₄₋₁₀ heterocycloalkylene -O-, and -C₄₋₁₀ heterocycloalkylene -S-.

According to an embodiment of the present disclosure, the linker is selected from the group consisting of -NH-C₄₋₈ alkylene-O-, -C₄₋₁₀ heterocycloalkylene-O-, and -C₄₋₁₀ heterocycloalkylene-(C₁₋₃ alkylene)-O- optionally substituted with hydroxy.

According to an embodiment of the present disclosure, the linker is selected from the group consisting of -NH-C₄₋₈ alkylene-O-, -C₄₋₈ heterocycloalkylene-O-, -C₄₋₈ heterocycloalkylene-CH₂-O- optionally substituted with hydroxy.

According to an embodiment of the present disclosure, the linker is selected from the group consisting of

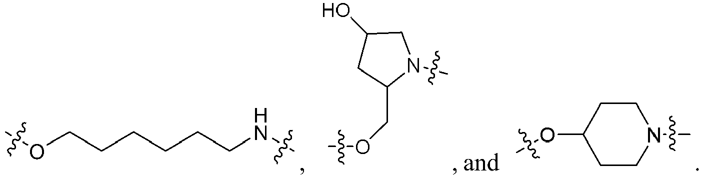

According to an embodiment of the present disclosure, the linker is selected from the group consisting of 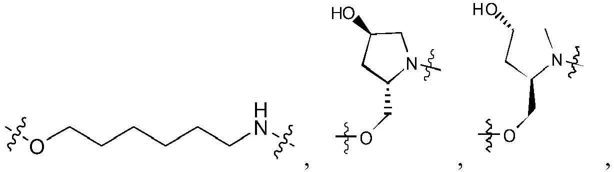 and

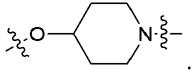

According to an embodiment of the present disclosure, R₁' is selected from the group consisting of -(C₀₋₁₀ alkylene)-C(O)-NH-(C₁₋₁₀ alkylene)-O-, -(C₀₋₁₀ alkylene)-C(O)-NH-(C₁₋₁₀ alkylene)-S-, -(C₀₋₁₀ alkylene)-C(O)-C₄₋₁₀ heterocycloalkylene -(C₁₋₆ alkylene)-O-, -(C₀₋₁₀ alkylene)-C(O)-C₄₋₁₀ heterocycloalkylene-(C₁₋₆ alkylene)-S-, -(C₀₋₁₀ alkylene)-C(O)-C₄₋₁₀ heterocycloalkylene-O-, and -(C₀₋₁₀ alkylene)-C(O)-C₄₋₁₀ heterocycloalkylene-S-. The C₄₋₁₀ heterocycloalkylene in -(C₀₋₁₀ alkylene)-C(O)-C₄₋₁₀ heterocycloalkylene-(C₁₋₆ alkylene)-O- and -(C₀₋₁₀ alkylene)-C(O)-C₄₋₁₀ heterocycloalkylene-(C₁₋₆ alkylene)-S- is optionally substituted with one or more -OH.

According to an embodiment of the present disclosure, R₁' is selected from the group consisting of 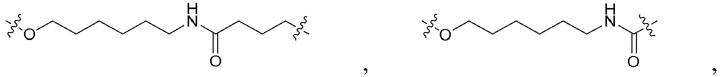 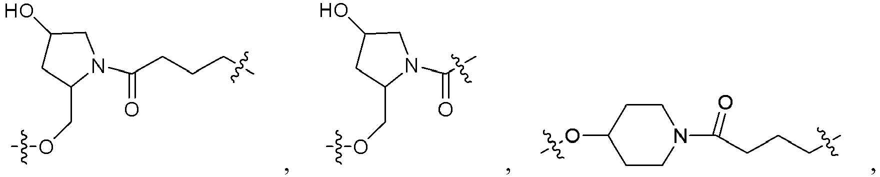 and

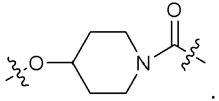

According to an embodiment of the present disclosure, R₁' is selected from the group consisting of 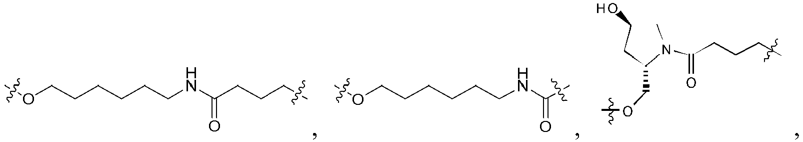 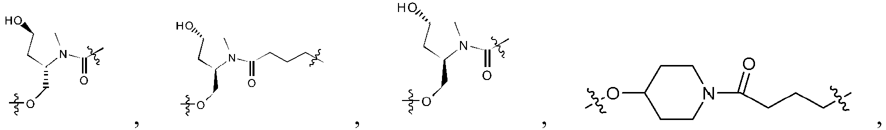 and

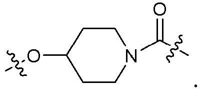

According to an embodiment of the present disclosure, the saccharide is glucose, lactose, galactose, or fructose.

According to an embodiment of the present disclosure, R₂ in G₁, G₂, G₃, and G₄ is each independently a residue of a compound selected from the group consisting of: N-acetylgalactosamine (GalNac), N-trifluoroacetylgalactosamine, N-formylgalactosamine, N-propionylgalactosamine, N-n-butyrylgalactosamine, and N-isobutyrylgalactosamine.

According to an embodiment of the present disclosure, R₂ in G₁, G₂, G₃, and G₄ is identical.

According to an embodiment of the present disclosure, G₁, G₂, G₃, and G₄ are each independently

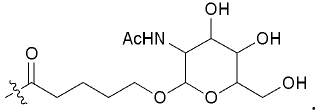

According to an embodiment of the present disclosure, G₁, G₂, G₃, and G₄ are each independently

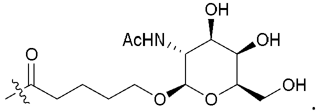

According to an embodiment of the present disclosure, the compound has a structure represented by formula (IIa): 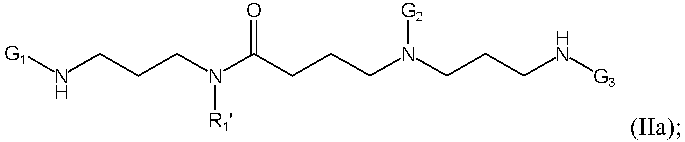
where: R₁' is 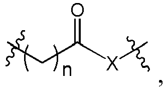 where n is any integer ranging from 1 to 6; and
G₁, G₂, G₃, and G₄ are as defined above.

According to an embodiment of the present disclosure, in formula (IIa), R₁' is selected from the group consisting of 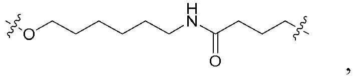 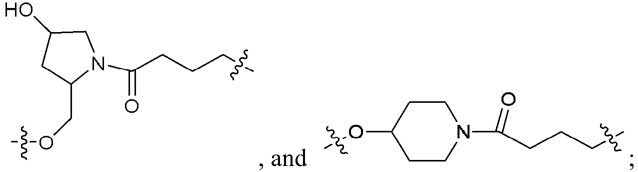 G₁, G₂, G₃, G₄ are each independently 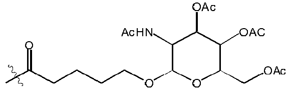 or

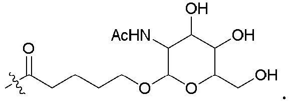

According to an embodiment of the present disclosure, in formula (IIa), R₁' is selected from the group consisting of 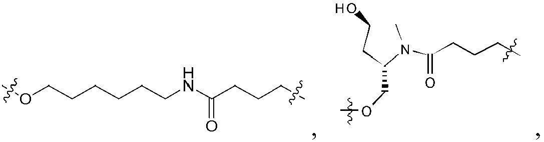 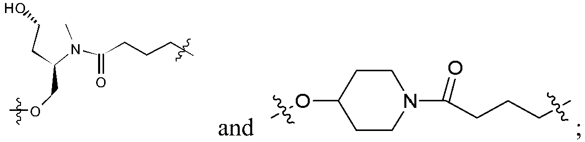 G₁, G₂, G₃, G₄ are each independently 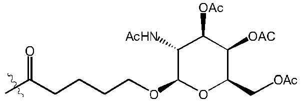 or

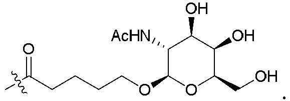

According to an embodiment of the present disclosure, the compound has a structure represented by formula (IIb): 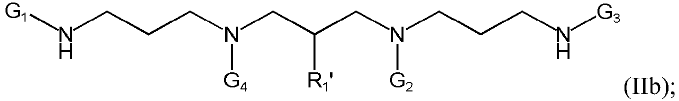
where: R₁' is 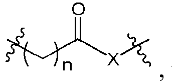 where n is 0; and
G₁, G₂, G₃, and G₄ are as defined above.

According to an embodiment of the present disclosure, in formula (IIb), R₁' is selected from the group consisting of 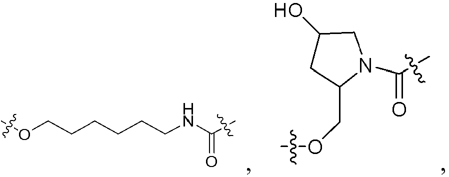 and 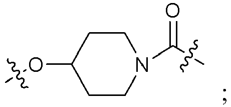 G₁, G₂, G₃, and G₄ are each independently 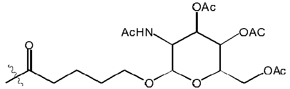 or

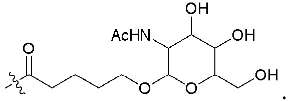

According to an embodiment of the present disclosure, in formula (IIb), R₁' is selected from the group consisting of 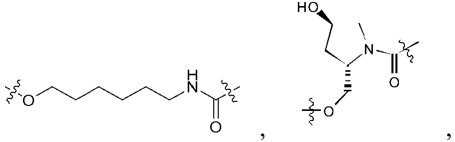 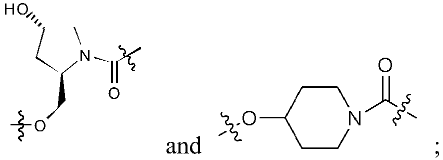 G₁, G₂, G₃, and G₄ are each independently 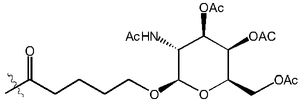 or

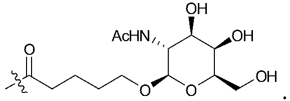

According to an embodiment of the present disclosure, the compound has any one of the following structures:

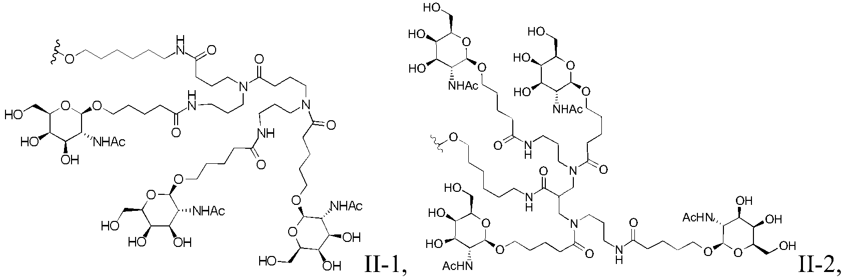

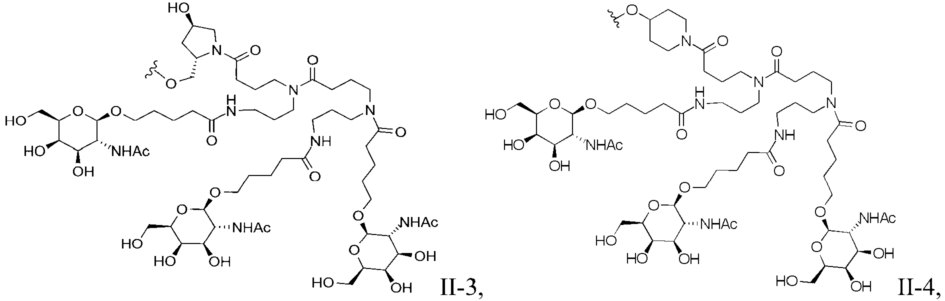

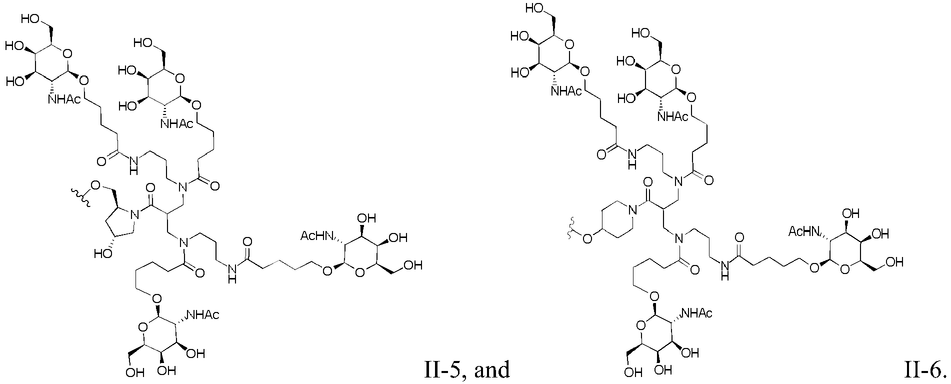

In a third aspect of the present disclosure, the present disclosure provides use of the compound or the stereoisomer, tautomer or pharmaceutically acceptable salt thereof according to the first aspect or the second aspect in the manufacture of a medicament.

According to an embodiment of the present disclosure, the medicament is used for targeting liver cells and/or renal cells.

According to an embodiment of the present disclosure, the medicament is used for targeted delivery of an active pharmaceutical agent to cells.

According to an embodiment of the present disclosure, the medicament is used for targeted delivery of an oligonucleotide to cells. In the present disclosure, the above-described compound, or the stereoisomer, tautomer or pharmaceutically acceptable salt thereof, serves as a targeting ligand and can be conjugated with a pharmaceutical agent such as an oligonucleotide for targeting and delivery of the nucleic acid pharmaceutical agent, enabling the nucleic acids to be delivered to desired cells and/or tissues in vivo (e.g., targeted delivery of an oligonucleotide for targeted silencing of a target gene in target cells and/or tissues), thus effectively preventing and/or treating the related diseases.

According to an embodiment of the present disclosure, the cells are preferably liver cells and/or renal cells.

According to an embodiment of the present disclosure, the medicament is used for reducing the expression or activity of a target gene.

In a fourth aspect of the present disclosure, the present disclosure further provides a conjugate. According to an embodiment of the present disclosure, the conjugate includes an active pharmaceutical agent and a ligand conjugated to the active pharmaceutical agent. The ligand is selected from the structure represented by formula (II) as defined in the compound or the stereoisomer, tautomer or pharmaceutically acceptable salt thereof according to the second aspect.

The present disclosure provides the conjugate. According to an embodiment of the present disclosure, the conjugate includes an oligonucleotide, and a ligand conjugated to the oligonucleotide. The ligand is selected from the structure represented by formula (II) as defined in the compound or the stereoisomer, tautomer or pharmaceutically acceptable salt thereof according to the second aspect.

According to an embodiment of the present disclosure, the above-described conjugate may further include at least one of the following technical features.

According to an embodiment of the present disclosure, the conjugate is obtained by reacting the active pharmaceutical agent (e.g., oligonucleotide) with the structure represented by formula (I) as defined in the compound or the stereoisomer, tautomer or pharmaceutically acceptable salt thereof according to the first aspect; or obtained by linking to the structure represented by formula (II) as defined in the compound or the stereoisomer, tautomer or pharmaceutically acceptable salt thereof according to the second aspect.

According to an embodiment of the present disclosure, the oligonucleotide includes siRNA, miRNA, or ASO.

In an optional embodiment of the present disclosure, the oligonucleotide is siRNA.

According to an embodiment of the present disclosure, the conjugate has one or more of the following features:
i) the ligand is linked to the sense strand and/or the antisense strand in siRNA;
ii) the ligand is linked to the sense strand and/or the antisense strand via a phosphorothioate bond or a phosphate bond;
iii) the ligand is linked to the 5' end or the 3' end of the sense strand; or
iv) the ligand is linked to the 5' end or the 3' end of the antisense strand.

According to an embodiment of the present disclosure, the ligand is linked to the oligonucleotide via the R1' group in the ligand.

According to an embodiment of the present disclosure, the ligand is linked to the oligonucleotide via the linker of the R1' group in the ligand.

According to an embodiment of the present disclosure, the conjugate includes a compound represented by formula (III), or the stereoisomer, tautomer or pharmaceutically acceptable salt thereof: 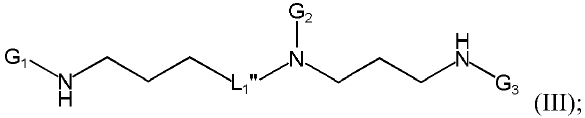
where: L₁" is -N(R₁")-C(O)-(C₁₋₁₀ alkylene)- or -N(G₄)-L₂"-;
L₂" is C₁₋₆ alkylene substituted with R₁";
G₁, G₂, G₃, and G₄ are each independently 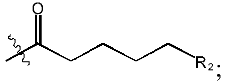
R₂ is a residue of a substituted or unsubstituted saccharide;
R₁" is -(C₀₋₁₀ alkylene)-C(O)-linker-R₁₂;
the linker is selected from the group consisting of -NH-(C₁₋₁₀ alkylene)-O-, -NH-(C₁₋₁₀ alkylene)-S-, -C₄₋₁₀ heterocycloalkylene-O-, -C₄₋₁₀ heterocycloalkylene-S-, -C₄₋₁₀ heterocycloalkylene-(C₁₋₆ alkylene)-O- optionally substituted with one or more Rₐ groups, and -C₄₋₁₀ heterocycloalkylene-(C₁₋₆ alkylene)-S- optionally substituted with one or more Rₐ groups, where the one or more Rₐ groups is each independently selected from -OH and -C₁₋₆ alkylene; and
R₁₂ is an active pharmaceutical agent, preferably an oligonucleotide.

According to an embodiment of the present disclosure, R₁₂ is siRNA.

According to an embodiment of the present disclosure, L₂" is C₂₋₄ alkylene substituted with R₁".

According to an embodiment of the present disclosure, L₂" is -CH₂-CH(R₁")-CH₂-.

According to an embodiment of the present disclosure, L₁" is -N(R₁")-C(O)-(C₁₋₆ alkylene)-.

According to an embodiment of the present disclosure, L₁" is -N(R₁")-C(O)-(C₁₋₆ alkylene)- and R₁" is -(C₁₋₆ alkylene)-C(O)-linker-R₁₂.

According to an embodiment of the present disclosure, L₁" is -N(G₄)-CH₂-CH(R₁")-CH₂-.

According to an embodiment of the present disclosure, L₁" is -N(G₄)-CH₂-CH (R₁")-CH₂- and R₁" is -C(O)-linker-R₁₂.

According to an embodiment of the present disclosure, R₁" is -(C₀₋₆ alkylene)-C(O)-R₁₂.

According to an embodiment of the present disclosure, the linker is selected from the group consisting of -NH-(C₁₋₁₀ alkylene)-O-, -NH-(C₁₋₁₀ alkylene)-S-, -C₄₋₁₀ heterocycloalkylene-O-, -C₄₋₁₀ heterocycloalkylene-S-, -C₄₋₁₀ heterocycloalkylene-(C₁₋₆ alkylene)-O- optionally substituted with one or more -OH, and -C₄₋₁₀ heterocycloalkylene-(C₁₋₆ alkylene)-S- optionally substituted with one or more -OH.

According to an embodiment of the present disclosure, the linker is -NH-(C₄₋₈ alkylene)-O-, -NH-(C₄₋₈ alkylene)-S-, -C₄₋₁₀ heterocycloalkylene-O-, -C₄₋₁₀ heterocycloalkylene-S-, -C₄₋₁₀ heterocycloalkylene-(C₁₋₃ alkylene)-O- substituted with one or more -OH, or -C₄₋₁₀ heterocycloalkylene-(C₁₋₃ alkylene)-S- substituted with one or more -OH.

According to an embodiment of the present disclosure, the linker is selected from the group consisting of -NH-(C₁₋₁₀ alkylene)-O-, -NH-(C₁₋₁₀ alkylene)-S-, -C₄₋₁₀ heterocycloalkylene-O-, and -C₄₋₁₀ heterocycloalkylene-S-.

According to an embodiment of the present disclosure, the linker is -NH-(C₄₋₈ alkylene)-O-, -C₄₋₁₀ heterocycloalkylene-O-, or -C₄₋₁₀ heterocycloalkylene-CH₂-O- optionally substituted with hydroxy.

According to an embodiment of the present disclosure, the linker is selected from the group consisting of

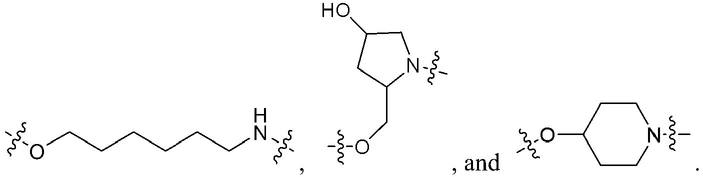

According to an embodiment of the present disclosure, the linker is selected from the group consisting of 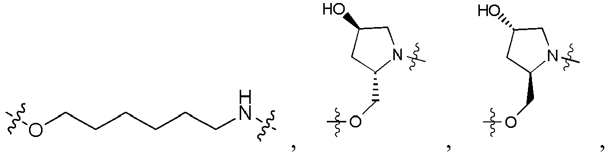 and

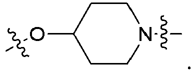

According to an embodiment of the present disclosure, R₁' is selected from the group consisting of -(C₀₋₁₀ alkylene)-C(O)-NH-(C₁₋₁₀ alkylene)-O-R₁₂, -(C₀₋₁₀ alkylene)-C(O)-NH-(C₁₋₁₀ alkylene)-S-R₁₂, -(C₀₋₁₀ alkylene)-C(O)-C₄₋₁₀ heterocycloalkylene -(C₁₋₆ alkylene)-O-R₁₂, -(C₀₋₁₀ alkylene)-C(O)-C₄₋₁₀ heterocycloalkylene-(C₁₋₆ alkylene)-S-R₁₂, -(C₀₋₁₀ alkylene)-C(O)-C₄₋₁₀ heterocycloalkylene-O-R₁₂, and -(C₀₋₁₀ alkylene)-C(O)-C₄₋₁₀ heterocycloalkylene-S-R₁₂. The C₄₋₁₀ heterocycloalkylene in -(C₀₋₁₀ alkylene)-C(O)-C₄₋₁₀ heterocycloalkylene-(C₁₋₆ alkylene)-O-R₁₂ and -(C₀₋₁₀ alkylene)-C(O)-C₄₋₁₀ heterocycloalkylene-(C₁₋₆ alkylene)-S-R₁₂ is optionally substituted with one or more -OH.

According to an embodiment of the present disclosure, R₁" is selected from the group consisting of 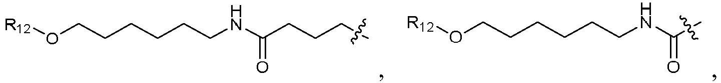 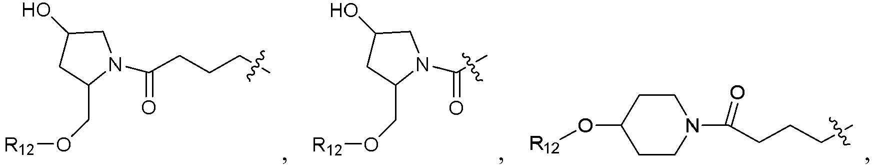 and

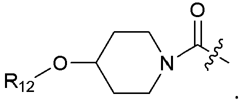

According to an embodiment of the present disclosure, R₁" is selected from the group consisting of

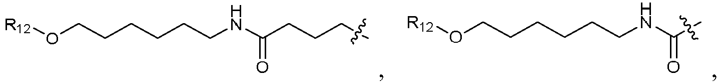

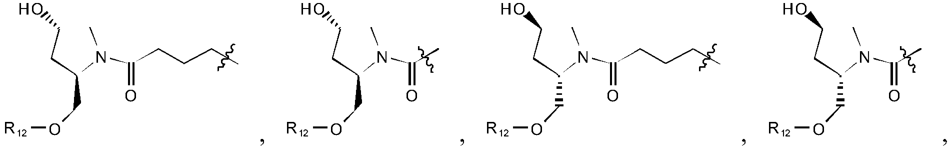

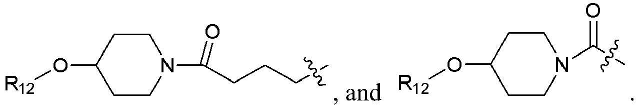

According to an embodiment of the present disclosure, R₁₂ is ASO, siRNA, or miRNA.

According to an embodiment of the present disclosure, the saccharide is glucose, lactose, galactose, or fructose.

According to an embodiment of the present disclosure, R₂ in G₁, G₂, G₃, and G₄ is each independently a residue of a compound selected from the group consisting of: N-acetylgalactosamine(GalNAc), N-trifluoroacetylgalactosamine, N-formylgalactosamine, N-propionylgalactosamine, N-n-butyrylgalactosamine, and N-isobutyrylgalactosamine.

According to an embodiment of the present disclosure, R₂ in G₁, G₂, G₃, and G₄ is identical.

According to an embodiment of the present disclosure, G₁, G₂, G₃, and G₄ are each independently

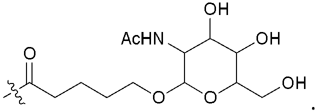

According to an embodiment of the present disclosure, G₁, G₂, G₃, and G₄ are each independently

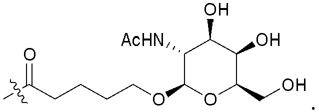

According to an embodiment of the present disclosure, the compound represented by formula (III) has a structure represented by formula (IIIa): 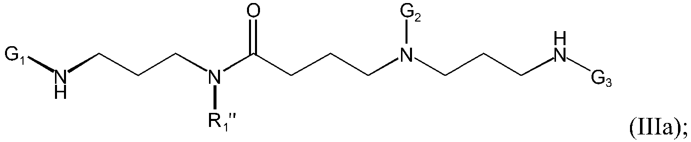
where: R₁" is -(C₁₋₆ alkylene)-C(O)-linker-R₁₂; and
G₁, G₂, G₃, and G₄ are as defined above.

According to an embodiment of the present disclosure, in formula (IIIa), R₁" is selected from the group consisting of 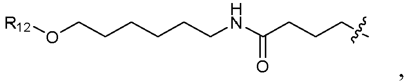 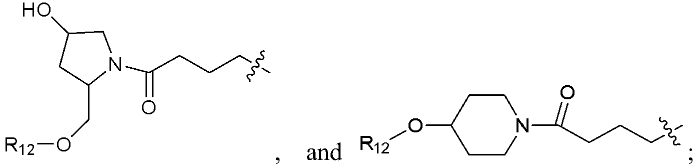 G₁, G₂, G₃, and G₄ are each independently 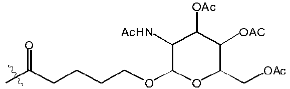 or

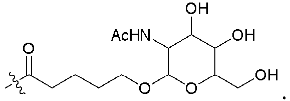

According to an embodiment of the present disclosure, in formula (IIIa), R₁" is selected from the group consisting of 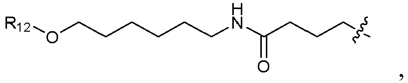 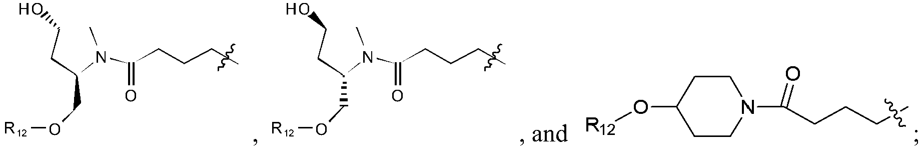 G₁, G₂, G₃, and G₄ are each independently 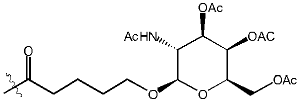 or

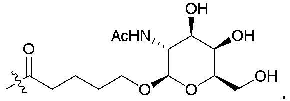

According to an embodiment of the present disclosure, the compound represented by formula (III) has a structure represented by formula (IIIb): 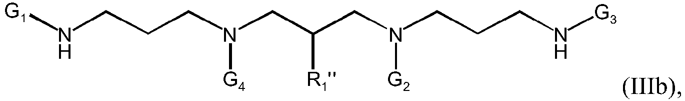
where: R₁" is -C(O)-linker-R₁₂; and
G₁, G₂, G₃, and G₄ are as defined above.

According to an embodiment of the present disclosure, in formula (IIIb), R₁" is selected from the group consisting of 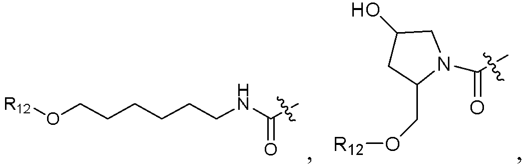 and 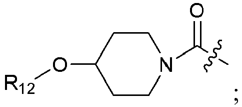 G₁, G₂, G₃, and G₄ are each independently 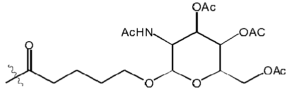 or

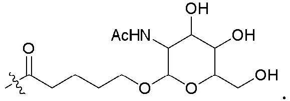

According to an embodiment of the present disclosure, in formula (IIIb), R₁"is selected from the group consisting of 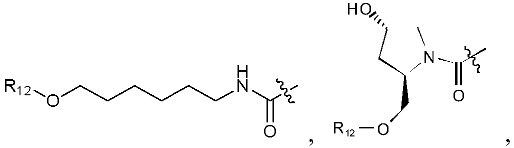 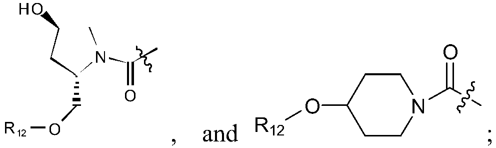 G₁, G₂, G₃, and G₄ are each independently 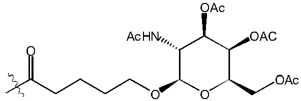 or

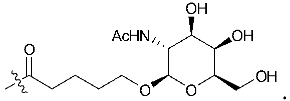

According to an embodiment of the present disclosure, the conjugate has any one of the following structures: 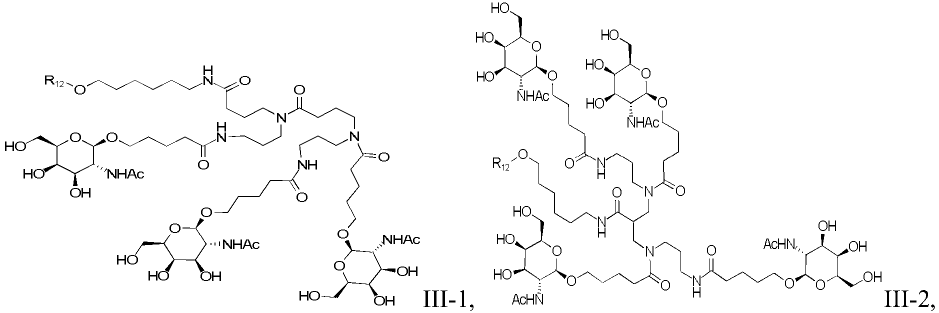 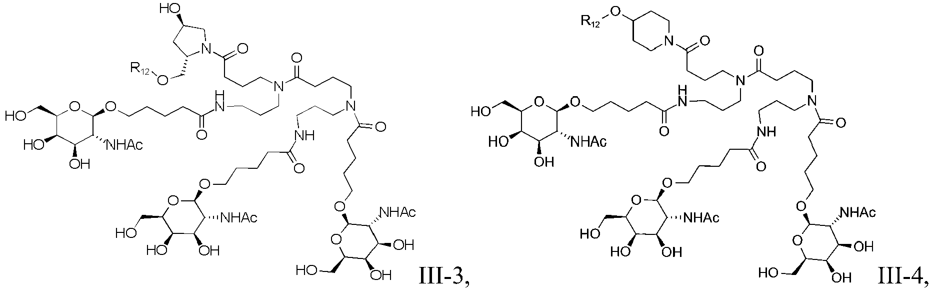 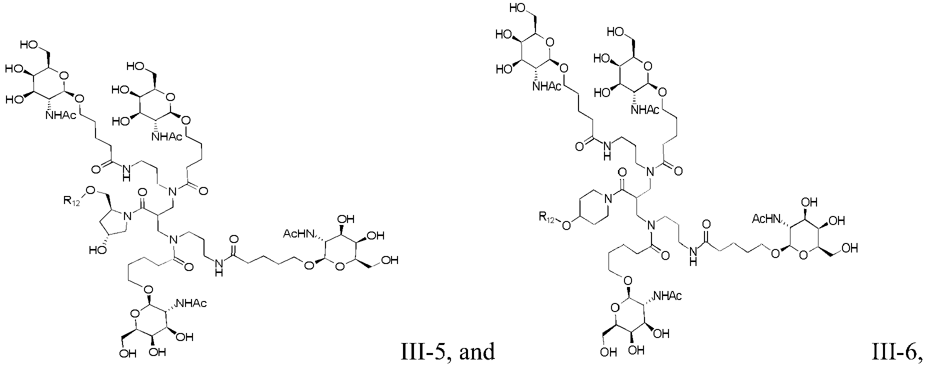 where R₁₂ is an active pharmaceutical agent, preferably an oligonucleotide.

According to an embodiment of the present disclosure, R₁₂ is ASO, siRNA, or miRNA.

According to an embodiment of the present disclosure, R₁₂ is siRNA.

In an optional embodiment of the present disclosure, R₁₂ is the sense strand and/or the antisense strand in siRNA.

In an optional embodiment of the present disclosure, R₁₂ is the sense strand in siRNA.

According to an embodiment of the present disclosure, the ligand is linked to the 3' end or the 5' end of the sense strand via a phosphorothioate bond or a phosphate bond.

Unless otherwise specified herein, in the sense strand and/or the antisense strand used for conjugating the ligand, the end of the sense strand and/or the antisense strand linked to the ligand includes a phosphorothioate bond or a phosphate bond, that is, the phosphorothioate bond or phosphate bond is located at the 3' end or the 5' end of the sense strand and/or the antisense strand and is used for conjugation with the ligand.

According to an embodiment of the present disclosure, the conjugate has any one of the following structures: 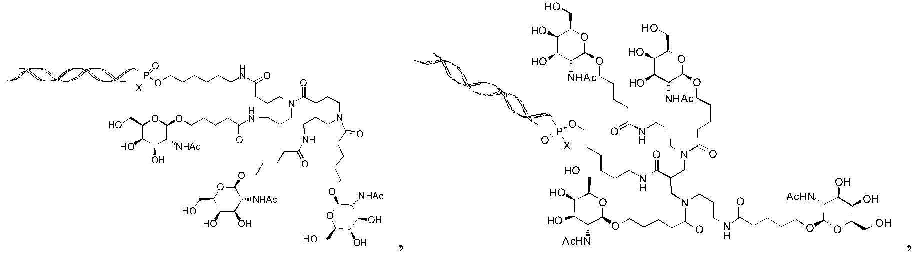 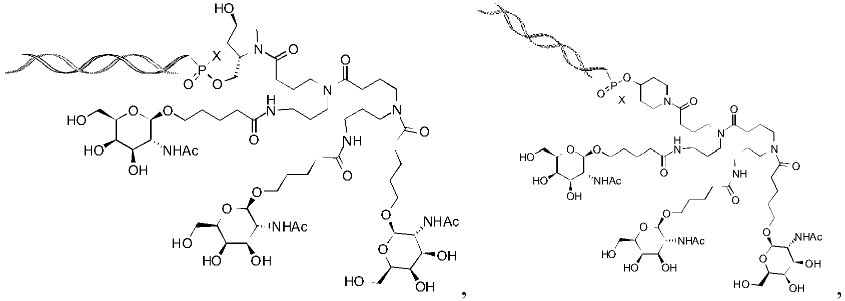 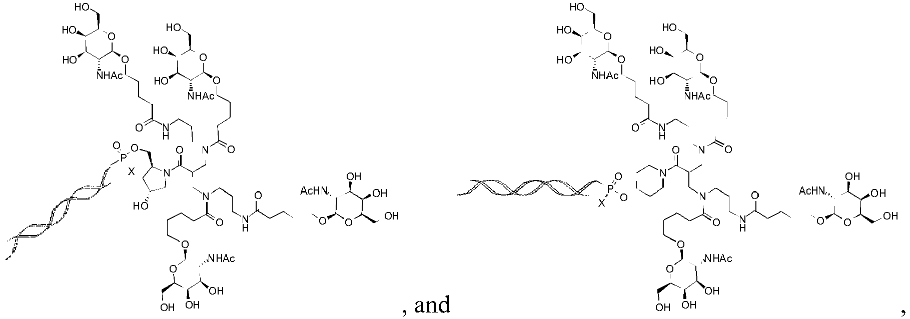 where 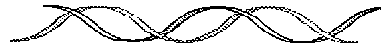 represents an oligonucleotide (e.g., R₁₂) and X represents O or S.

In a fifth aspect of the present disclosure, the present disclosure provides a pharmaceutical composition. According to an embodiment of the present disclosure, the pharmaceutical composition includes the compound or the stereoisomer, tautomer or pharmaceutically acceptable salt thereof according to the second aspect, or the conjugate according to the fourth aspect.

According to an embodiment of the present disclosure, the pharmaceutical composition further includes a pharmaceutically acceptable carrier or excipient.

In a sixth aspect of the present disclosure, the present disclosure provides a method for targeting cells. According to an embodiment of the present disclosure, the method includes introducing the conjugate according to the fourth aspect into the cells.

In the present disclosure, the above-described compound, or the stereoisomer, tautomer or pharmaceutically acceptable salt thereof, serves as a targeting ligand and can be conjugated with an active pharmaceutical agent such as an oligonucleotide for targeting and delivery of the active pharmaceutical agent, enabling the active pharmaceutical agent to enter desired cells and/or tissues in vivo and/or in vitro (e.g., targeted delivery of an oligonucleotide for targeted silencing of a target gene in target cells and/or tissues).

According to an embodiment of the present disclosure, the cells are liver cells and/or renal cells.

In a seventh aspect of the present disclosure, the present disclosure provides use of the conjugate according to the fourth aspect or the pharmaceutical composition according to the fifth aspect in: the manufacture of a medicament for preventing and/or treating a disease; the prevention and/or treatment of a disease; the reduction of expression or activity of a target gene; or targeted delivery of an active pharmaceutical agent to cells. The cells are preferably liver cells and/or renal cells.

The conjugate of the present disclosure, by means of the compound or the stereoisomer, tautomer or pharmaceutically acceptable salt thereof, can be used for targeting and delivery of an active pharmaceutical agent, enabling the active pharmaceutical agent (e.g., the oligonucleotide/nucleic acids of the present disclosure) to be delivered to desired cells and/or tissues in vivo (e.g., targeted delivery of the oligonucleotide for targeted silencing of a target gene in target cells and/or tissues), thus effectively preventing and/or treating the related diseases.

In an eighth aspect of the present disclosure, the present disclosure provides the conjugate according to the fourth aspect or the pharmaceutical composition according to the fifth aspect for use in the prevention and/or treatment of a disease, and/or for use in the manufacture of a medicament for reducing expression or activity of a target gene, and/or for use in the targeted delivery of an active pharmaceutical agent to cells. The cells are preferably liver cells and/or renal cells.

The conjugate of the present disclosure, by means of the compound or the stereoisomer, tautomer or pharmaceutically acceptable salt thereof, can be used for targeting and delivery of an active pharmaceutical agent, enabling the active pharmaceutical agent (e.g., the oligonucleotide/nucleic acids of the present disclosure) to be delivered to desired cells and/or tissues in vivo (e.g., targeted delivery of the oligonucleotide for targeted silencing of a target gene in target cells and/or tissues), thus effectively preventing and/or treating the related diseases.

In a ninth aspect of the present disclosure, the present disclosure provides a method for preventing and/or treating a disease. According to an embodiment of the present disclosure, the method includes administering to a subject a pharmaceutically acceptable amount of the conjugate according to the fourth aspect or the pharmaceutical composition according to the fifth aspect.

An effective amount of the conjugate or the pharmaceutical composition of the present disclosure may vary depending on factors such as the mode of administration and the severity of the disease to be treated. The selection of a preferred effective amount may be determined by those skilled in the art based on various factors (e.g., through clinical trials). Such factors include, but are not limited to, pharmacokinetic parameters of the active ingredient such as bioavailability, metabolism, or half-life, the severity of the disease to be treated in the patient, the body weight of the patient, the immune status of the patient, and the route of administration.

Additional aspects and advantages of the present disclosure will be provided at least in part in the following description, or will become apparent at least in part from the following description, or may be learned from practicing of the present disclosure.

### BRIEF DESCRIPTION OF THE DRAWINGS

The above and/or additional aspects and advantages of the present disclosure will become more apparent and readily understood from the following description of embodiments in conjunction with the accompanying drawings.

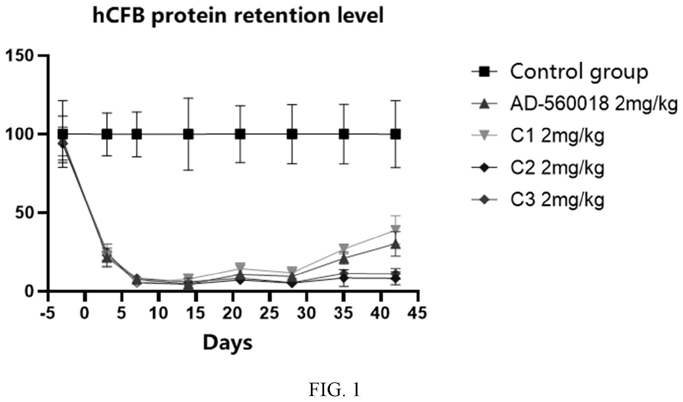

FIG. 1 shows the retained hCFB protein levels in Tg-hCFB mice in vivo after administration of the siRNA conjugate in Test Example 3 of the present disclosure.

### DETAILED DESCRIPTION OF THE EMBODIMENTS

Embodiments of the present disclosure will be described in detail below. The embodiments described below are illustrative only, and are intended to explain, rather than limit, the present disclosure.

It should be noted that, the terms "first" and "second" are only used for descriptive purposes, and cannot be understood as indicating or implying relative importance or implicitly indicating the number of indicated technical features. Therefore, the features associated with "first" and "second" may explicitly or implicitly include at least one of the features. Further, in the description of the present disclosure, "plurality" means at least two, unless otherwise specifically defined.

As used in the present disclosure, the terms "comprising" or "including" are openended expressions and are intended to cover the components explicitly mentioned as well as additional elements not expressly recited.

As used in the present disclosure, the terms "optionally," "optional," or "option" mean that the subsequently described event or circumstance may or may not occur, and that the description includes both instances where the event or circumstance occurs and where it does not.

As used herein, the term "small interfering RNA (siRNA)" refers to a double-stranded RNA including a sense strand and an antisense strand. siRNA, through formation of an RNA-induced silencing complex (RISC), mediates targeted cleavage of RNA transcripts via the RISC pathway. Specifically, siRNA directs specific degradation of mRNA sequences through the RNA interference (RNAi) process, thus inhibiting translation of mRNA into amino acids and subsequent conversion into proteins.

As used herein, the term "antisense strand" (also referred to as "guide strand") includes a region substantially complementary to a target sequence, such as an mRNA encoding a target protein. The term "sense strand" (also referred to as "passenger strand") refers to a strand that includes a sequence substantially complementary to the antisense strand of the iRNA. The term "substantially complementary" means fully complementary or at least partially complementary. For example, the antisense strand is fully complementary or at least partially complementary to the target sequence. In the case of partial complementarity, mismatches may be present in internal or terminal regions of a molecule. The most tolerated mismatches are present in the terminal regions, e.g., within 5, 4, 3, or 2 nucleotides at the 5' end and/or the 3' end of iRNA.

It should be noted that "at least partially substantially complementary" between the antisense strand and the mRNA means that the antisense strand includes a polynucleotide that is substantially complementary to a contiguous portion of the mRNA of interest (e.g., an mRNA encoding a target protein). Alternatively, if a polynucleotide is substantially complementary in a substantially uninterrupted manner to a portion of an mRNA encoding a target protein, then the antisense strand is complementary to at least a portion of the mRNA encoding the target protein.

As used herein, the term "target sequence" and "target nucleotide sequence" both refer to a contiguous portion of the nucleotide sequence of an mRNA molecule formed during transcription of a target gene, including mRNA that is an RNA processing product of a primary transcript.

It should be noted that, with respect to structural formulas and chemical formulas illustrated in examples or embodiments of the present disclosure, the present disclosure is intended to cover all the alternatives, modifications, and equivalent technical solutions, which are all within the scope of the present disclosure as defined by the claims. Those skilled in the art should understand that many methods and materials similar or equivalent to those described herein can be used to practice the present disclosure. The present disclosure is by no means limited to the methods and materials described herein. In the event that one or more of the incorporated documents, patents, and similar materials differ from or contradict the present disclosure (including but not limited to defined terms, application of terms, described techniques, etc.), the present disclosure shall control.

It should be further understood that certain features of the present disclosure, which are for clarity described in a plurality of separate embodiments, may also be provided in combination in a single example or embodiment. Conversely, various features of the present disclosure, which are for brevity described in a single example or embodiment, may also be provided separately or in any suitable sub-combination.

Unless otherwise indicated, the technical and scientific terms used in the present disclosure have the same meanings as commonly understood by those skilled in the art to which the present disclosure belongs. Unless otherwise indicated, all the patent publications cited in the entire disclosure of the present disclosure are incorporated herein by reference in entirety.

The present disclosure employs the following definitions unless otherwise indicated. For the objectives of the present disclosure, chemical elements are defined in accordance with the Periodic Table of the Elements, CAS version, Handbook of Chemicals, 75th Ed., 1994. In addition, general principles of organic chemistry are described in "Organic Chemistry", Thomas Sorrell, University Science Books, Sausalito: 1999, and "March's Advanced Organic Chemistry", by Michael B. Smith and Jerry March, John Wiley & Sons, New York: 2007. Therefore, all the contents of the present disclosure incorporate such references.

The compounds of the present disclosure may contain asymmetric centers or chiral centers and thus exist in different stereoisomeric forms. It is intended that all stereoisomeric forms of the compounds of the present disclosure, including but not limited to diastereomers, enantiomers, and atropisomers, and mixtures of such stereoisomers such as racemic mixtures, are included within the scope of the present disclosure. Many organic compounds exist in optically active forms, that is, such compounds have an ability to rotate a plane of plane-polarized light. When describing an optically active compound, prefixes D and L or R and S are used to represent an absolute configuration of a molecule with respect to a chiral center (or chiral centers) in the molecule. Prefixes d and l or (+) and (-) are used to designate a sign of rotation of a plane-polarized light caused by a compound, where (-) or l indicates that a compound is levorotatory. A compound with the prefix (+) or d is dextrorotatory. For a given chemical structure, the stereoisomers are identical except that the stereoisomers are mirror images of each other. A specific stereoisomer may also be called an enantiomer, and mixtures of the isomers are generally referred to as mixtures of enantiomers. A 50:50 mixture of the enantiomers is referred to as a racemic mixture or a racemate. The racemic mixture or racemate may occur when no stereoselectivity or stereospecificity is present in a chemical reaction or a method.

Based on selection of raw materials and methods, the compounds of the present disclosure may exist in a form of one possible isomer or a mixture of possible isomers, for example, as pure optical isomers, or as isomer mixtures such as racemic mixtures and diastereomeric mixtures, depending on the number of asymmetric carbon atoms. Optically active (R)- or (S)-isomers may be prepared using chiral synthons or chiral reagents, or resolved using conventional techniques. If the compound contains one double bond, substituents may be of E or Z configuration. If the compound contains a disubstituted cycloalkyl group, substituents on the cycloalkyl group may be of cis- or trans-configuration.

The compounds of the present disclosure may contain asymmetric centers or chiral centers and thus exist in different stereoisomeric forms. It is intended that all the stereoisomeric forms of the compounds of the present disclosure, including but not limited to diastereomers, enantiomers, atropisomers, and geometric (or conformational) isomers, and mixtures of these isomers such as racemic mixtures, are within the scope of the present disclosure.

Unless otherwise indicated, a structure described in the present disclosure also includes all the isomeric forms of the structure (e.g., enantiomers, diastereomeric atropisomers, and geometric (or conformational) forms), for example, the R and S configurations at each asymmetric center, (Z) and (E) double bond isomers, and (Z) and (E) conformational isomers. Therefore, individual stereochemical isomers of the compounds of the present disclosure, mixtures of enantiomers, mixtures of diastereomers, and mixtures of geometric (or conformational) isomers are all within the scope of the present disclosure.

Any asymmetric atom (such as carbon) of the compounds of the present disclosure may exist in a racemic form or in an enantiomerically enriched form, for example in (R)-, (S)- or (R, S)-configuration. In some embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess with respect to (R)- or (S)-configuration. If possible, substituents on atoms having an unsaturated double bond may exist in a cis-(Z)- form or a trans-(E)-form.

Therefore, as described in the present disclosure, the compounds of the present disclosure may exist in one form or a mixture of possible isomers, rotamers, atropisomers, tautomers, for example, in a form of substantially pure geometric (cis or trans) isomers, diastereoisomers, optical isomers (enantiomers), racemates, or mixtures thereof.

Any mixture of isomers obtained may be separated into pure or substantially pure geometric or optical isomers, diastereomers, and racemates based on physicochemical differences of components, for example, by chromatography and/or fractional crystallization.

Racemates of any obtained final product or intermediate may be resolved into optical enantiomers by methods known to those skilled in the art, for example, by separation of diastereomeric salts obtained therefrom. Racemic products may also be separated by chiral chromatography, for example, by high pressure liquid chromatography (HPLC) using chiral adsorbents. In particular, the enantiomers may be prepared by asymmetric synthesis.

As used herein, the term "tautomer" or "tautomeric form" refers to a structural isomer having different energies that are interconvertible via a low energy barrier. If tautomerization is possible (for example, in a solution), chemical equilibrium of the tautomer may be achieved. For example, a protontautomer (also referred to as a prototropic tautomer) includes interconversion via migration of a proton, such as keto-enol isomerization and imine-enamine isomerization. A valence tautomer includes an interconversion via reorganization of some bonding electrons. Unless otherwise indicated, all the tautomeric forms of the compounds of the present disclosure are within the scope of the present disclosure.

As used herein, the term "pharmaceutically acceptable salt" refers to an organic salt and an inorganic salt of the compounds of the present disclosure. The pharmaceutically acceptable salt is well known in the art, as described in the literature: S. M. Berge et al., describe pharmaceutically acceptable salts in detail in J. Pharmaceutical Sciences, 1977, 66: 1-19.

" 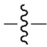 " or " 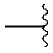 " in the description of a group in the present disclosure is used to indicate a substitution position of the group.

In the chemical structures of the ligand or the compounds described in the present disclosure, the bond "-" indicates that the configuration is unspecified. If chiral isomerism exists in a chemical structure, the bond "-" may be " 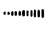 " or " 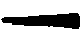 ", or include both " 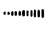 " and " 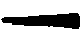 " configurations. Although all the structural formulas described above are drawn as certain isomeric forms for simplicity, the present disclosure may include all the isomers, for example: tautomers, rotamers, geometric isomers, diastereomers, racemates, and enantiomers.

As used herein, the terms "optionally substituted with", "optionally substituted by", and "substituted or unsubstituted" may be used interchangeably. Generally, the term "optionally", whether preceding the term "substituted" or not, indicates that one or more hydrogen atoms in a given structure are substituted with a specified substituent. Unless otherwise indicated, an optionally substituted group may be substituted at each substitutable position of the group. When more than one position in a given structural formula can be substituted with one or more substituents selected from the specified group, the substituent may be substituted at each position, identically or differently. The substituent includes, but is not limited to, F, Cl, Br, CN, OH, NH₂, NO₂, etc.

As used herein, the term "one or more" (for example, in the definition of substituents of the compounds of the general formula of the present disclosure) refers to "one, two, three, four, or five", particularly "one, two, three, or four", more particularly "one, two, or three", even more particularly "one or two".

In addition, it should be noted that, unless explicitly indicated otherwise, expressions "each ... is independently", "... are each independently", and "... is independently" used in the present disclosure are interchangeable and should be understood in a broad sense. These expressions mean that specific options represented by a same symbol are independent of each other, either in different groups or in a same group.

As used herein, the term "halogen" refers to fluorine, chlorine, bromine, or iodine atom.

As used herein, a minimum value and a maximum value of carbon atom content in a hydrocarbon group are indicated by a prefix. For example, a prefix C_{a-b} refers to containing "a" to "b" carbon atoms. Exemplarily, "C₁₋ₙ" refers to a straight or branched, saturated/unsaturated carbon chain containing 1, 2, 3, 4, 5, ..., or n carbon atoms. It should be further understood that, "C₁₋ₙ" should be interpreted as including any sub-range included therein, such as C₁₋₁₀, C₁₋₆, etc. C₁₋₆ includes C₁₋₅, C₁₋₄, C₁₋₃, C₁₋₂, C₂₋₅, C₂₋₄, C₂₋₃, C₃₋₅, C₃₋₄, and C₄₋₅.

It should be noted that the term "C₁₋₁₀" as used herein, for example, in the context of the definition of "C₁₋₁₀ alkyl", refers to alkyl having a limited number of carbon atoms ranging from 1 to 10, i.e., 1, 2, 3, 4, 5, 6, 7, 8, 9, or 10 carbon atoms. It should be further understood that, the term "C₁₋₁₀" should be interpreted as including any sub-range included therein, such as C₁₋₆, C₂₋₅, C₃₋₄, C₁₋₂, C₁₋₃, C₁₋₄, and C₁₋₅; particularly C₁₋₂, C₁₋₃, C₁₋₄, C₁₋₅, and C₁₋₆; and more particularly C₂₋₆.

Similarly, as used herein, the term "C₂₋₆" used throughout the specification, for example, in the context of the definitions of "C₂₋₆ alkenyl" and "C₂₋₆ alkynyl", should be understood to refer to alkenyl or alkynyl having a limited number of carbon atoms ranging from 2 to 6, i.e., 2, 3, 4, 5 or 6 carbon atoms. It should be further understood that, the term "C₂₋₆" should be interpreted as including any sub-range included therein, for example, C₂₋₆, C₃₋₅, C₃₋₄, C₂₋₃, C₂₋₄, and C₂₋₅; and particularly C₂₋₃.

As used herein, the term "C₁₋₁₀ alkyl" refers to a straight or branched saturated monovalent hydrocarbon group having 1, 2, 3, 4, 5, ..., or 10 carbon atoms, for example, C₁₋₆ alkyl, C₁₋₅ alkyl, C₁₋₄ alkyl, C₁₋₃ alkyl, C₂₋₅ alkyl, C₂₋₄ alkyl, and C₂₋₃ alkyl. Such alkyl includes, but is not limited to, methyl, ethyl, n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, - CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), and 3,3-dimethyl-2-butyl (-CH(CH₃)C(CH₃)₃). The alkyl group may be independently unsubstituted or substituted with one or more substituents as described in the present disclosure.

As used herein, the term "alkylene" refers to a group formed by removal of one further hydrogen atom from "alkyl". "C₁₋₆ alkylene " includes methylene, ethylene, propylene, isopropylene (e.g. 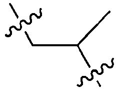 ), butylene (e.g. 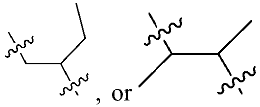 ), pentylene (e.g. 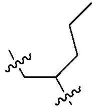 ,), hexylene (e.g. 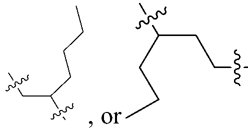 ), etc.

As used herein, the terms "heterocycloalkyl", "heterocycle", and "heterocycloalkane" each refer to a saturated or non-aromatic unsaturated ring containing at least one heteroatom. The heteroatom refers to a nitrogen atom, an oxygen atom, a sulfur atom, etc. The terms generally represent a monovalent saturated or partially unsaturated monocyclic or bicyclic ring system having a plurality of ring atoms, which contains 1, 2, or 3 ring heteroatoms selected from N, O, and S, with the remaining ring atoms being carbon.

The 4- to 10-membered heterocycle described in the present disclosure refers to a 4-, 5-, 6-, ..., or 10-membered saturated or unsaturated heterocycle. The term "unsaturated" means that a group or a molecule contains a carbon-carbon double bond, carbon-carbon triple bond, carbon-oxygen double bond, carbon-sulfur double bond, carbon-nitrogen triple bond, etc.

As used herein, "pharmaceutical composition" may refer to a composition used for treatment of a disease or for in vitro cell culture experiments. When used for the treatment of a disease, the term "pharmaceutical composition" generally refers to a pharmaceutical agent in unit-dose form and may be prepared by any method well known in the pharmaceutical field. All methods include a step for combining an active ingredient with excipients that constitute one or more auxiliary components. Typically, the composition is prepared by uniformly and thoroughly combining active siRNA with a liquid excipient, a finely divided solid excipient, or both the liquid excipient and the finely divided solid excipient.

As used herein, the term "pharmaceutically acceptable" means that a substance or a composition must be compatible chemically and/or toxicologically with other ingredients of a formulation and/or a mammal treated therewith. Preferably, "pharmaceutically acceptable" described in the present disclosure refers to substances approved by a federal regulatory agency or a national government, or listed in the U.S. Pharmacopeia or other generally recognized pharmacopeia for use in animals, particularly in humans.

As used herein, the term "pharmaceutically acceptable excipient" includes any solvent, diluent, or other liquid vehicle, etc., suitable for a specific target dosage form. Use of conventional excipients is also encompassed within the scope of the present disclosure, excluding those excipients that are incompatible with the siRNA of the present disclosure, such as those that produce any adverse biological effect or interact in a harmful manner with any other component of the pharmaceutically acceptable composition.

The use of the conventional excipients is also encompassed within the scope of the present disclosure, excluding those excipients that are incompatible with the siRNA of the present disclosure, such as those that produce any adverse biological effect or interact in a harmful manner with any other component of the pharmaceutically acceptable composition.

As used herein, the term "treatment" refers to obtaining of a desired pharmacological and/or physiological effect. The effect may be prophylactic in terms of complete or partial prevention of a disease or symptoms thereof, and/or therapeutic in terms of partial or complete cure of a disease and/or adverse effects caused by the disease. "Treatment" as used herein covers diseases in mammals, particularly in humans, and includes: (a) prevention of a disease or condition in an individual that is susceptible to the disease but not yet diagnosed as having the disease; (b) inhibition of a disease, for example, arrest of disease progression; or (c) alleviation of a disease, for example, mitigation of symptoms associated with the disease. "Treatment" as used herein includes any administration of a pharmaceutical agent or siRNA to an individual to treat, cure, alleviate, ameliorate, reduce, or inhibit a disease in the individual, including but not limited to administration of a pharmaceutical agent including the siRNA or a siRNA conjugate described herein to an individual in need thereof.

### Detailed Description of a Targeting Compound for Nucleic Acid Delivery and Uses Thereof in the Present Disclosure

The present disclosure provides a compound represented by formula (I) or formula (II) or a stereoisomer, tautomer or pharmaceutically acceptable salt thereof, a conjugate, a pharmaceutical composition, a method for targeting a protein, and related uses, each of which is described in detail below.

### Compound

The present disclosure provides a compound represented by formula (I), or a stereoisomer, tautomer or pharmaceutically acceptable salt thereof: 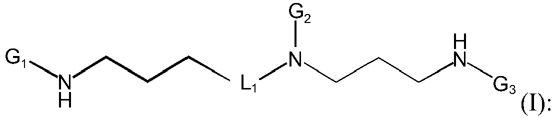
where: L₁ is -N(R₁)-C(O)-(C₁₋₁₀ alkylene)- or -N(G₄)-L₂-;
L₂ is C₁₋₆ alkylene substituted with R₁;
G₁, G₂, G₃, and G₄ are each independently 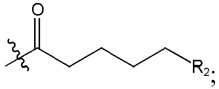
R₂ is a residue of a substituted or unsubstituted saccharide;
R₁ is -(C₀₋₁₀ alkylene)-C(O)-R₁₀; and
R₁₀ is a leaving group.

As used herein, "leaving group" refers to a group in the compound represented by formula (I) that can be removed upon reaction with other compounds.

According to an embodiment of the present disclosure, substituents in R₂ include, but are not limited to, protecting groups. The protecting groups are preferably those used to avoid destruction of a group (such as hydroxyl group) in a compound during a reaction. The protecting groups include, but are not limited to, 4,4'-dimethoxytrityl (DMTr) or acetyl (Ac).

As used herein, the term "residue" means a group obtained by removal of one H from the above-described saccharide.

It should be noted that partial definitions for each group in the compound represented by formula (I) refer to the second aspect of the summary in this specification.

### Ligand

As used herein, the term "ligand" or "targeting ligand" refer to a class of substances capable of altering distribution, targeting, or lifetime of an oligonucleotide. For example, provision of a ligand can enhance the affinity of the oligonucleotide for a selected target (such as an mRNA encoding CFB) compared with an oligonucleotide without the ligand. The ligand may be a natural protein (such as human serum albumin (HSA)), a carbohydrate (such as dextran and chitosan) or a lipid. The ligand may also be a recombinant or synthetic molecule, such as a synthetic polymer. Preferably, the ligand does not participate in pairing of the sense strand and the antisense strand in the oligonucleotide.

The present disclosure provides a ligand, which is a compound represented by formula (II) of the present disclosure, or a stereoisomer, tautomer or pharmaceutically acceptable salt thereof: 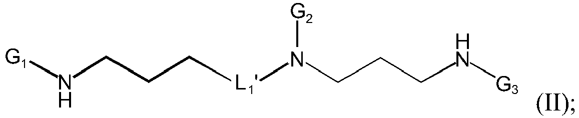
where: L₁' is -N(R₁')-C(O)-(C₁₋₁₀ alkylene)- or -N(G₄)-L₂'-;
L₂' is C₁₋₆ alkylene substituted with R₁';
G₁, G₂, G₃, and G₄ are each independently 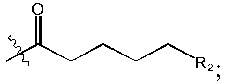
R₂ is a residue of a substituted or unsubstituted saccharide;
R₁' is 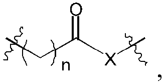 where n is any integer ranging from 0 to 10; and
X is absent or a linker;
the linker, if present, is -NH-(C₁₋₁₀ alkylene)-O-, -NH-(C₁₋₁₀ alkylene)-S-, -C₄₋₁₀ heterocycloalkylene-(C₁₋₆ alkylene)-O- optionally substituted with one or more Rₐ groups, -C₄₋₁₀ heterocycloalkylene-(C₁₋₆ alkylene)-S- optionally substituted with one or more Rₐ groups, - C₄₋₁₀ heterocycloalkylene-O-, or -C₄₋₁₀ heterocycloalkylene-S-, where the one or more Rₐ groups is each independently selected from -OH and -C₁₋₆ alkylene. Preferably, X is a linker.

In an optional embodiment of the present disclosure, partial definitions of each group in the compound represented by formula (II) refer to the third aspect of the summary in this specification.

### Use

The present disclosure provides use of the compound or the stereoisomer, tautomer or pharmaceutically acceptable salt thereof according to the first aspect or the second aspect in the manufacture of a medicament for targeting liver cells and/or renal cells.

The compound of the present disclosure, or the stereoisomer, tautomer or pharmaceutically acceptable salt thereof serves as a targeting ligand and can be conjugated with a pharmaceutical agent such as an oligonucleotide for targeting and delivery of a nucleic acid pharmaceutical agent, enabling nucleic acids to be delivered to desired cells and/or tissues in vivo (e.g., targeted delivery of the oligonucleotide for targeted silencing of a target gene in target cells and/or tissues), thus effectively preventing and/or treating related diseases.

According to an embodiment of the present disclosure, the pharmaceutical agent further includes an oligonucleotide.

According to an embodiment of the present disclosure, the oligonucleotide is conjugated to the compound or the stereoisomer, tautomer or pharmaceutically acceptable salt thereof according to the second aspect; and/or
the oligonucleotide is obtained by reaction with a structure represented by formula (I) as defined in the compound or the stereoisomer, tautomer or pharmaceutically acceptable salt thereof according to the first aspect.

According to an embodiment of the present disclosure, the oligonucleotide includes siRNA, miRNA, or ASO.

### Nucleic Acid Conjugate

When an active pharmaceutical agent is a nucleic acid/oligonucleotide, the present disclosure provides a nucleic acid conjugate, which includes an oligonucleotide, and a ligand conjugated to the oligonucleotide. The ligand has a structure represented by formula (II) as defined in the compound or the stereoisomer, tautomer or pharmaceutically acceptable salt thereof according to the third aspect of the summary the present disclosure. The ligand is conjugated to the oligonucleotide (such as siRNA) either directly or indirectly via a linker. By incorporating the ligand, the nucleic acid conjugate of the present disclosure enables further targeted delivery of the oligonucleotide to target cells (for example, improving degradation of mRNA encoding a target protein by the oligonucleotide), thus effectively preventing and/or treating the related diseases.

As used herein, the oligonucleotide may be selected based on a disease type or the target protein without particular limitations, and all such oligonucleotides are within the protection scope of the present disclosure.

The linkage manner between the ligand and siRNA is also not particularly limited. The ligand may be directly linked to siRNA via a group without using an additional linker/linking group, or indirectly linked to siRNA via a linker.

The linkage manner between the ligand and the sense strand and/or the antisense strand of siRNA is also not particularly limited. Preferably, the ligand is linked to siRNA via a phosphorothioate bond or a phosphate bond. Preferably, the ligand is linked to the 5'-end or the 3'-end of the sense strand. Preferably, the ligand is linked to the 5'-end or the 3'-end of the antisense strand.

According to an embodiment of the present disclosure, the ligand has a structure shown as II-1, II-2, II-3, II-4, II-5, or II-6 of the ligand according to the second aspect, which are correspondingly named compound P7, P8, P9, P10, P11, or P12.

Therefore, the use of the above-described ligand can facilitate targeting and delivery of the oligonucleotide, enabling the oligonucleotide to be delivered to desired cells and/or tissues in vivo, facilitating targeted silencing of a target gene in target cells and/or tissues, thus effectively preventing and/or treating the related diseases.

As used herein, unless otherwise specified, the oligonucleotide is not particularly limited and may be siRNA (such as the siRNA described in the first aspect of the present disclosure), miRNA, or ASO.

According to an embodiment of the present disclosure, the oligonucleotide is synthesized to have an NH₂-alkyl group (such as an NH₂-C₆ alkylene group) at the 5'-end of the sense strand of siRNA. The NH₂-alkyl group includes a partial linker structure (such as - NH-(C₁₋₁₀ alkylene)) in the ligand structure according to the second aspect. That is, upon linkage with a ligand precursor of the present disclosure (i.e., the compound represented by formula (I) or the stereoisomer, tautomer or pharmaceutically acceptable salt thereof according to the first aspect of the present disclosure), the NH₂-alkyl group constitutes the partial linker structure (such as -NH-(C₁₋₁₀ alkylene)) in the ligand structure according to the second aspect. In some embodiments, an oligonucleotide has the NH₂-alkyl group, and the terminal amino group of the NH₂-alkyl group may subsequently be conjugated with the ligand represented by formula (II) according to the second aspect of the present disclosure to form a nucleic acid conjugate. In some embodiments, a phosphate group or a phosphorothioate group of an oligonucleotide reacts with the compound represented by formula (I) or the stereoisomer, tautomer or pharmaceutically acceptable salt thereof according to the first aspect of the present disclosure, to form a nucleic acid conjugate. In some embodiments, the oligonucleotide provided in the present disclosure is synthesized to have one or more alkynyl groups at the 5'-end of the sense strand of siRNA. The terminal alkynyl group may subsequently react with a group of the ligand of the present disclosure to form a conjugate.

As used herein, when an oligonucleotide of the nucleic acid conjugate is siRNA, the siRNA includes a sense strand and an antisense strand, and a phosphorothioate bond or a phosphate bond for linking to a linker in the ligand structure according to the third aspect. As described above, the NH₂-alkyl group mentioned above belongs to the ligand. The NH₂-alkyl group is pre-linked to the sense strand of siRNA to facilitate synthesis of the nucleic acid conjugate of the present disclosure.

### Linker

In some embodiments, when an active group R₁ in the ligand precursor of the present disclosure (i.e., the compound represented by formula (I) or the stereoisomer, tautomer or pharmaceutically acceptable salt thereof according to first aspect of the present disclosure) contains no linker, the active group R₁ in the ligand may be conjugated with/linked to the oligonucleotide directly, or conjugated with/linked to the oligonucleotide via the linker (linking group). The linker of the present disclosure is not particularly limited. In some embodiments, when the conjugate includes two or more oligonucleotides, the two or more oligonucleotides may be linked to the active group R₁ of the ligand via a same linker or different linkers. According to an embodiment of the present disclosure, the manner of conjugating the linker to the oligonucleotide is not particularly limited. The linker may be linked to the 3'-end and/or the 5'-end of the sense strand of the oligonucleotide, or to the 3'-end and/or the 5'-end of the antisense strand.

Examples of the linker include, but are not limited to: -NH-C₆ alkylene-S-S-C₆ alkylene-O-, active groups such as primary amines (for example, having any one of the following structures: 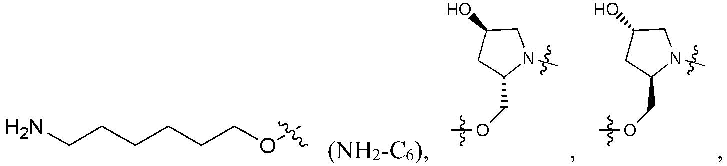 and 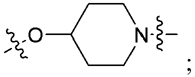 and a structure after linking the oligonucleotide to the ligand is 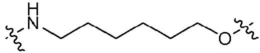 ), alkynes, alkenes, alkyl groups, abasic residues/nucleotides, amino acids, triyne-functionalized groups, ribitol, and/or PEG groups.

In an optional embodiment of the present disclosure, the linker, after being linked to the phosphate group or the phosphorothioate group of the oligonucleotide, has any one of the following structures: 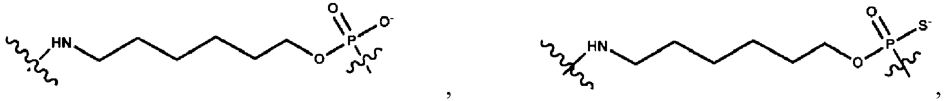 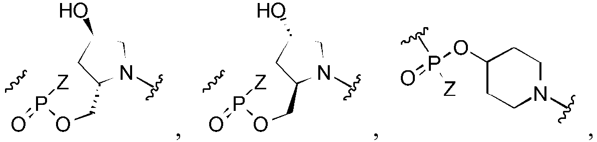 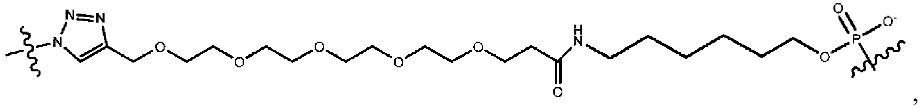 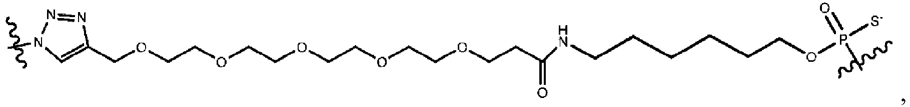 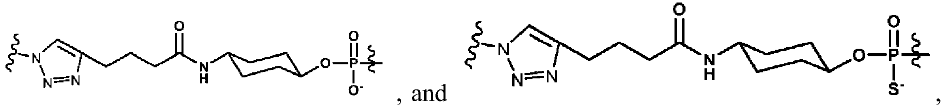 where Z is O or S.

### Method for preventing and/or treating a disease

The present disclosure provides a method for preventing and/or treating a disease. According to an embodiment of the present disclosure, the method includes administering to a subject a pharmaceutically acceptable amount of the conjugate according to the fourth aspect or the pharmaceutical composition according to the fifth aspect.

An effective amount of the conjugate or the pharmaceutical composition of the present disclosure may vary depending on factors such as the mode of administration and the severity of the disease to be treated. The selection of a preferred effective amount may be determined by those skilled in the art based on various factors (e.g., through clinical trials). Such factors include, but are not limited to, pharmacokinetic parameters of the active ingredient such as bioavailability, metabolism, or half-life, the severity of the disease to be treated in the patient, the body weight of the patient, the immune status of the patient, and the route of administration.

The solution of the present disclosure is explained below in conjunction with the examples. It will be appreciated by those skilled in the art that the following examples are only used to illustrate the present disclosure and should not be constructed as limiting the scope of the present disclosure. Where specific techniques or conditions are not indicated in the examples, they are carried out in accordance with the techniques or conditions described in the literature in the art or in accordance with the product manuals. The reagents or instruments used without specifying the manufacturers are all conventional products that can be purchased commercially.

### Preparation Example 1: Preparation of Ligand I-1 to Ligand I-3

### 1. Preparation of Ligand 1-1

A synthetic route is as follows:

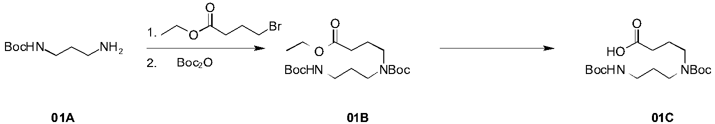

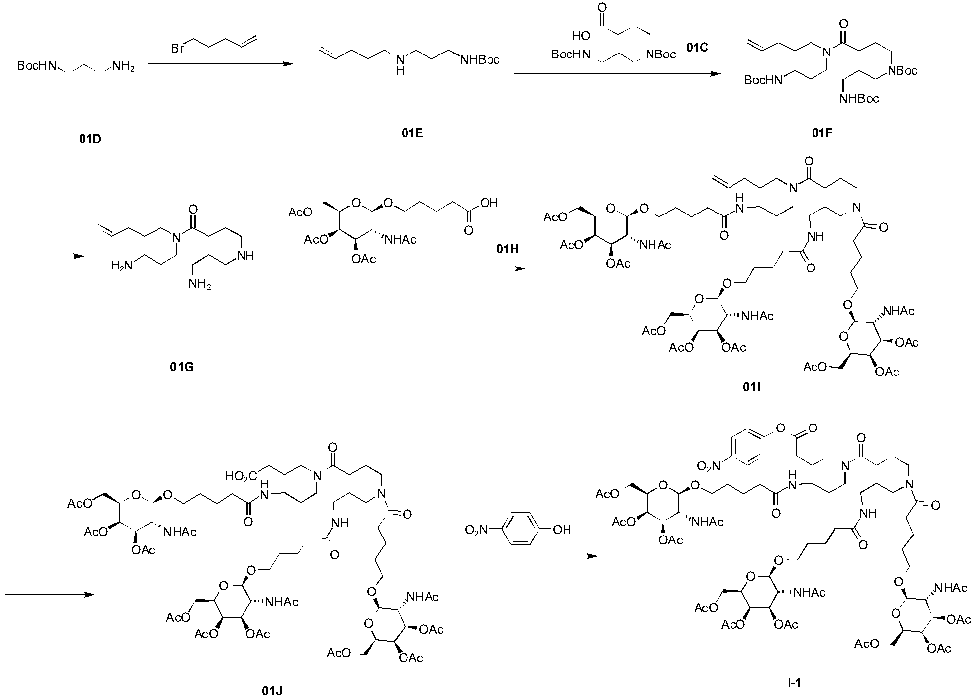

### Step 1 : Synthesis of ethyl 4-((tert-butoxycarbonyl)(3-((tert-butoxycarbonyl)amino)propyl)amino)butanoate (01B):

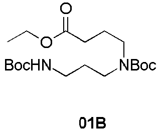

N-(Tert-butoxycarbonyl)-1,3-propanediamine (30.00 g, 172.2 mmol), acetonitrile (250 mL), and potassium carbonate (47.59 g, 344.3 mmol) were sequentially added to a 500 mL single-necked flask. The mixture was cooled to 0°C, and ethyl 4-bromobutyrate (26.87 g, 137.8 mmol) was added dropwise slowly. After the dropwise addition was completed, the mixture was warmed naturally to a room temperature and reacted for 16 hours. The reaction mixture was filtered, and di-tert-butyl dicarbonate (56.36 g, 258.2 mmol) was added to the filtrate, followed by reaction at room temperature for 5 hours. The reaction mixture was concentrated under reduced pressure to obtain a crude product, which was purified by column chromatography (petroleum ether/ethyl acetate (v/v) = 1:0 to 7:3) to obtain ethyl 4-((tert-butoxycarbonyl)(3-((tert-butoxycarbonyl)amino)propyl)amino)butanoate (01B) (45.00 g, yield 84.08%).

LC-MS, M/Z (ESI): 289.4 [M-99]⁺

### Step 2: Synthesis of 4-((tert-butoxycarbonyl)(3-((tert-butoxycarbonyl)amino)propyl)amino)butanoic acid (01C):

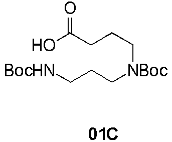

Ethyl 4-((tert-butoxycarbonyl)(3-((tert-butoxycarbonyl)amino)propyl)amino) butanoate (45.00 g, 115.8 mmol), methanol (200 mL), and an aqueous solution (50 mL) of lithium hydroxide (5.55 g, 231.8 mmol) were sequentially added to a 500 mL single-necked flask. The mixture was stirred at room temperature for 16 hours. The reaction solution was concentrated under reduced pressure. Water (50 mL) was added, and 1 N hydrochloric acid was added to adjust the pH value to 6. Ethyl acetate (150 mL×3) was added for extraction. Combined organic phase was washed with saturated brine, dried over anhydrous sodium sulfate, and filtered. The mixture was concentrated under reduced pressure to obtain 4-((tert-butoxycarbonyl)(3-((tert-butoxycarbonyl)amino)propyl)amino)butanoic acid (01C) (24.00 g, yield 57.49%).

¹H NMR (400 MHz, DMSO-*d*₆) δ 6.73 (s, 1H), 3.08 (q, *J* = 8.6, 8.1 Hz, 4H), 2.85 (q, *J =* 6.6 Hz, 2H), 2.12 (t, *J=* 7.3 Hz, 2H), 1.67 - 1.60 (m, 2H), 1.57 - 1.49 (m, 2H), 1.36 (s, 9H), 1.35 (s, 9H).

LC-MS, M/Z (ESI): 383.4 [M+Na]⁺.

### Step 3: Synthesis of tert-butyl (3-(pent-4-en-1-ylamino)propyl)carbamate (01E):

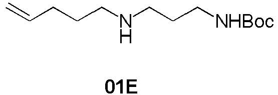

N-(Tert-butoxycarbonyl)-1,3-propanediamine (30.00 g, 172.2 mmol), acetonitrile (250 mL), and potassium carbonate (47.59 g, 344.3 mmol) were sequentially added to a 500 mL single-necked flask. The mixture was cooled to 0°C, and 5-bromo-1-pentene (20.53 g, 137.8 mmol) was added dropwise slowly. After the dropwise addition was completed, the mixture was warmed naturally to room temperature and stirred for 16 hours. The reaction solution was filtered and concentrated under reduced pressure to obtain a crude product, which was purified by column chromatography (dichloromethane/methanol (V/V) = 1:0 to 20:1) to obtain tert-butyl (3-(pent-4-en-1-ylamino)propyl)carbamate (01E) (16.00 g, yield 38.34%).

¹H NMR (400 MHz, DMSO-*d*₆) δ 6.78 (t, *J =* 5.0 Hz, 1H), 5.84 - 5.74 (m, 1H), 5.00 - 4.95 (m, 1H), 4.93 - 4.89 (m, 1H), 3.14 (s, 1H), 2.92 (q, *J* = 6.7 Hz, 2H), 2.43 (t, *J* = 7.0 Hz, 4H), 2.01 (q, *J=* 7.3 Hz, 2H), 1.49 - 1.40 (m, 4H), 1.34 (s, 9H).

LC-MS, M/Z (ESI): 243.3 [M+H]⁺

### Step 4: Synthesis of tert-butyl (3-((tert-butoxycarbonyl)amino)propyl)(4-((3-((tert-butoxycarbonyl)amino)propyl)(pent-4-en-1-yl)amino)-4-oxobutyl)carbamate (01F):

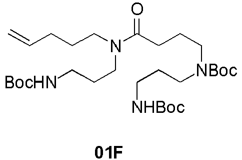

Tert-butyl (3-(pent-4-en-1-ylamino)propyl)carbamate (16.00 g, 66.02 mmol), dichloromethane (200 mL), 4-((tert-butoxycarbonyl)(3-((tert-butoxycarbonyl)amino)propyl)amino)butanoic acid (23.80 g, 66.03 mmol), 1-ethyl-3-(3-dimethylaminopropyl)carbodiimide hydrochloride (18.98 g, 99.01 mmol), 1-hydroxybenzotriazole (8.92 g, 66.01 mmol), and triethylamine (20.04 g, 198.0 mmol) were sequentially added to a 500 mL single-necked flask. The mixture was reacted at room temperature for 16 hours under nitrogen protection. The reaction mixture was washed sequentially with 0.5 N hydrochloric acid (100 mL) and saturated aqueous sodium bicarbonate solution (100 mL), dried over anhydrous sodium sulfate, filtered, and concentrated under reduced pressure to obtain a crude product. The crude product was purified by column chromatography (petroleum ether/ethyl acetate (V/V) = 1:0 to 0:1) to obtain tert-butyl (3-((tert-butoxycarbonyl)amino)propyl)(4-((3-((tert-butoxycarbonyl)amino)propyl)(pent-4-en-1-yl)amino)-4-oxobutyl)carbamate (01F) (19.00 g, yield 49.21%).

¹H NMR (400 MHz, DMSO-*d*₆) δ 6.91 - 6.60 (m, 2H), 5.84 - 5.72 (m, 1H), 5.06 - 4.89 (m, 2H), 3.22 - 3.14 (m, 4H), 3.12 - 3.06 (m, 4H), 2.90 - 2.83 (m, 4H), 2.23 - 2.10 (m, 2H), 1.98 - 1.92 (m, 2H), 1.47 - 1.66 (m, 8H), 1.35 (d, *J=* 3.8 Hz, 27H).

LC-MS, M/Z (ESI): 607.6 [M+Na]⁺.

### Step 5: Synthesis of N-(3-aminopropyl)-4-((3-aminopropyl)amino)-N-(pent-4-en-1-yl)butanamide trifluoroacetate (01G):

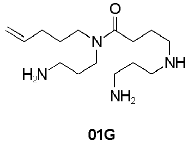

Tert-butyl(3-((tert-butoxycarbonyl)amino)propyl)(4-((3-((tert-butoxycarbonyl)amino)propyl)(pent-4-en-1-yl)amino)-4-oxobutyl)carbamate (5.00 g, 8.55 mmol), dichloromethane (200 mL), and trifluoroacetic acid (13.09 mL, 171.1 mmol) were added to a 250 mL single-necked flask. The mixture was stirred at room temperature for 16 hours. The reaction solution was concentrated under reduced pressure to obtain N-(3-aminopropyl)-4-((3-aminopropyl)amino)-N-(pent-4-en-1-yl)butanamide trifluoroacetate (01G) (5.34 g, yield 99.7%).

LC-MS, M/Z (ESI): 285.46 [M+H]⁺

### Step 6: Synthesis of (2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-5,11,20-trioxo-10-(pent-4-en-1-yl)-6,10,15,19-tetraazaicosane-1,24-diyl)bis(oxy))bis(5-acetamido-2-(acetoxymethyl)tetrahydro-2H-pyran-6,3,4-triyl) tetraacetate (01I):

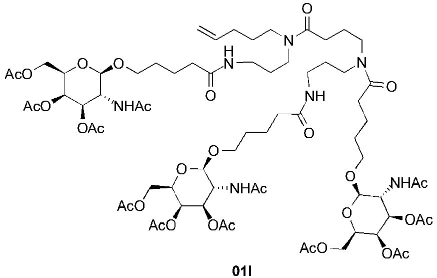

N-(3-aminopropyl)-4-((3-aminopropyl)amino)-N-(pent-4-en-1-yl)butanamide trifluoroacetate (01G) (3.30 g, 5.27 mmol), dichloromethane (100 mL), 5-(((2R,3R,4R,5R,6R)-3-acetamido-4,5-diacetoxy-6-(acetoxymethyl)tetrahydro-2H-pyran-2-yl)oxy)pentanoic acid (8.26 g, 18.5 mmol), 1-ethyl-3-(3-dimethylaminopropyl)carbodiimide hydrochloride (5.05 g, 26.3 mmol), 1-hydroxybenzotriazole (2.14 g, 15.8 mmol), and triethylamine (5.34 g, 52.8 mmol) were sequentially added to a 250 mL single-necked flask. The mixture was stirred at room temperature for 16 hours under nitrogen protection. The reaction mixture was washed sequentially with 0.5 N hydrochloric acid (100 mL) and saturated aqueous sodium bicarbonate solution (100 mL), dried over anhydrous sodium sulfate, filtered, and concentrated under reduced pressure to obtain a crude product. The crude product was purified by column chromatography (dichloromethane/methanol (V/V) = 1:0 to 9:1) to obtain (2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-5,11,20-trioxo-10-(pent-4-en-1-yl)-6,10,15,19-tetraazaicosane-1,24-diyl)bis(oxy))bis(5-acetamido-2-(acetoxymethyl)tetrahydro-2H-pyran-6,3,4-triyl) tetraacetate (7.00 g, yield 84.5%).

¹H NMR (600 MHz, DMSO-*d*₆) δ 7.85 - 7.77 (m, 4H), 7.71 - 7.69 (m, 1H), 5.83 - 5.77 (m, 1H), 5.19 (d, *J* = 3.3 Hz, 3H), 5.05 - 4.91 (m, 5H), 4.46 (dd, *J* = 8.4, 4.1 Hz, 3H), 4.02 - 3.99 (m, 9H), 3.89 - 3.82 (m, 3H), 3.70 - 3.68 (m, 3H), 3.40 - 3.37 (m, 3H), 3.25 - 3.16 (m, 8H), 3.04 - 2.95 (m, 4H), 2.30 - 2.17 (m, 4H), 2.08 (s, 9H), 2.05 - 1.94 (m, 15H), 1.87 (s, 9H), 1.75 (s, 9H), 1.61 - 1.43 (m, 20H).

LC-MS, M/Z (ESI): 787.26 [M+2H]²⁺

### Step 7: Synthesis of 4-(4-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)butanamido)butanoic acid (01J):

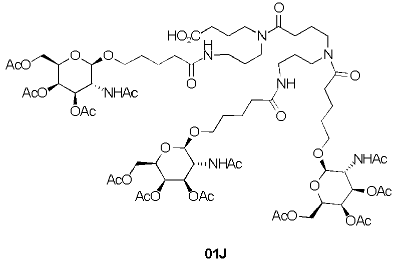

(2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-5,11,20-trioxo-10-(pent-4-en-1-yl)-6,10,15,19-tetraazaicosane-1,24-diyl)bis(oxy))bis(5-acetamido-2-(acetoxymethyl)tetrahydro-2H-pyran-6,3,4-triyl) tetraacetate (7.00 g, 4.45 mmol), dichloromethane (30 mL), acetonitrile (30 mL), and water (48 mL) were sequentially added to a 250 mL single-necked flask and stirred uniformly. Sodium periodate (4.76 g, 22.3 mmol) and ruthenium trichloride (18.5 mg, 89.0 µmol) were then added. The mixture was stirred at room temperature for 16 hours under nitrogen protection. The reaction solution was filtered. Saturated aqueous sodium bicarbonate solution was added to the filtrate to adjust the pH value of the aqueous phase to 7 to 8. After liquid separation, the aqueous phase was obtained, washed with dichloromethane (50 mL×2), adjusted to a pH value of about 5 to 6 with 1 N hydrochloric acid, and extracted by adding dichloromethane (100 mL×3). The obtained organic phase was dried over anhydrous sodium sulfate, filtered, and concentrated under reduced pressure to obtain 4-(4-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)butanamido)butanoic acid (01J) (3.50 g, yield 49.4%).

¹H NMR (600 MHz, DMSO-*d*₆) δ 12.12 (s, 1H), 7.84 - 7.81 (m, 4H), 7.74 - 7.71 (m, 1H), 5.19 (d, *J* = 3.2 Hz, 3H), 4.94 (dd, *J* = 11.2, 3.3 Hz, 3H), 4.47 (d, *J* = 8.4 Hz, 3H), 4.00 - 3.99 (m, 9H), 3.88 - 3.83 (m, 3H), 3.71 - 3.66 (m, 3H), 3.40 - 3.37 (m, 3H), 3.22 - 3.17 (m, 8H), 3.04 - 2.95 (m, 4H), 2.30 - 2.13 (m, 6H), 2.08 (s, 9H), 2.04 - 2.01 (m, 4H), 1.98 (s, 9H), 1.87 (s, 9H), 1.75 (s, 9H), 1.69 - 1.43 (m, 20H).

LC-MS, M/Z(ESI): 796.60 [M+2H]²⁺

### Step 8: Synthesis of (2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-10-(4-(4-nitrophenoxy)-4-oxobutyl)-5,11,20-trioxo-6,10,15,19-tetraazaicosane-1,24-diyl)bis(oxy))bis(5-acetamido-2-(acetoxymethyl)tetrahydro-2H-pyran-6,3,4-triyl) tetraacetate (I-1):

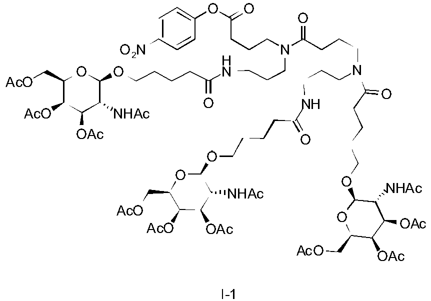

4-(4-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)butanamido)butanoic acid (900 mg, 0.566 mmol), dichloromethane (20 mL), 4-nitrophenol (94.5 mg, 0.679 mmol), and N,N'-dicyclohexylcarbodiimide (175.11 mg, 0.85 mmol) were sequentially added to a 100 mL single-necked flask. The mixture was stirred at room temperature for 16 hours under nitrogen protection. The reaction mixture was filtered, and the filtrate was concentrated to obtain a crude product, which was purified by column chromatography (dichloromethane/methanol (V/V) = 1:0 to 9:1) to obtain (2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-10-(4-(4-nitrophenoxy)-4-oxobutyl)-5,11,20-trioxo-6,10,15,19-tetraazaicosane-1,24-diyl)bis(oxy))bis(5-acetamido-2-(acetoxymethyl)tetrahydro-2H-pyran-6,3,4-triyl) tetraacetate (I-1) (850 mg, yield 87.8%).

¹H NMR (400 MHz, DMSO-*d*₆) δ 8.29 (dd, *J* = 9.2, 2.7 Hz, 2H), 7.82 - 7.67 (m, 4H), 7.74 - 7.66 (m, 1H), 7.44 (dd, *J* = 9.1, 2.1 Hz, 2H), 5.19 (d, *J=* 3.3 Hz, 3H), 4.94 (dd, *J* = 11.2, 3.4 Hz, 3H), 4.46 (d, *J* = 8.4 Hz, 3H), 4.00 (s, 9H), 3.85 (q, *J* = 9.2 Hz, 3H), 3.72 - 3.65 (m, 3H), 3.41 - 3.37 (m, 3H), 3.24 - 3.19 (m, 8H), 3.06 - 2.92 (m, 4H), 2.77 - 2.49 (m, 2H), 2.36 - 2.15 (m, 4H), 2.07 - 2.08 (m, 9H), 2.03 - 2.02 (m, 4H), 1.97 (s, 9H), 1.87 (s, 9H), 1.75 (s, 9H), 1.67 - 1.37 (m, 20H).

LC-MS, M/Z (ESI): 856.5 [M+2H]²⁺

### 2. Preparation of ligand I-2

A synthetic route is as follows:

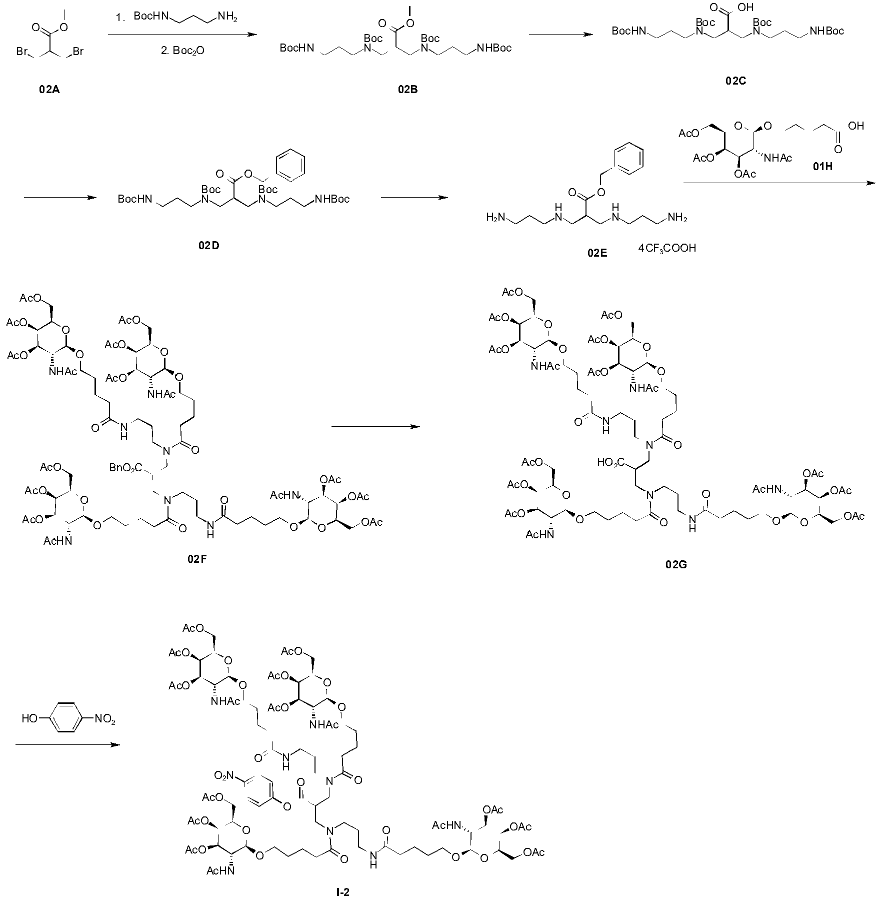

### Step 1 : Synthesis of methyl 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoate (02B):

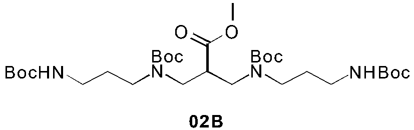

Methyl 3-bromo-2-(bromomethyl)propanoate (2.00 g, 7.69 mmol), N-(tert-butoxycarbonyl)-1,3-propanediamine (5.36 g, 30.8 mmol), and acetonitrile (30 mL) were sequentially added to a 100 mL single-necked flask and stirred at room temperature for 16 hours. The reaction mixture was concentrated under reduced pressure. Dichloromethane (30 mL), di-tert-butyl dicarbonate (6.72 g, 30.8 mmol), and N,N-diisopropylethylamine (3.98 g, 30.8 mmol) were added, and the mixture was stirred at room temperature for 16 hours. 50 mL water was added to the reaction mixture, which was then extracted with dichloromethane (50 mL×3). The combined organic phase was washed with saturated brine, dried over anhydrous sodium sulfate, filtered, and the filtrate was concentrated under reduced pressure to obtain a crude product, which was purified by column chromatography (petroleum ether/ethyl acetate (V/V) = 1:0 to 3:2) to obtain methyl 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoate (02B) (3.50 g, yield 70.7%).

### Step 2: Synthesis of 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoic acid (02C):

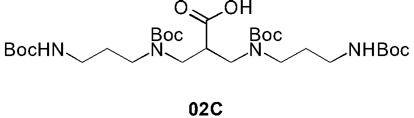

Methyl 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoate (3.50 g, 5.44 mmol), methanol (30 mL), and an aqueous solution (10 mL) of lithium hydroxide (389 mg, 16.2 mmol) were sequentially added to a 100 mL single-necked flask and stirred at room temperature for 16 hours. The mixture was concentrated under reduced pressure to remove organic solvent. 50 mL water was added. The pH value was adjusted to 6 with 1 N hydrochloric acid, and the mixture was extracted with ethyl acetate (150 mL×3). The combined organic phase was washed with saturated brine, dried over anhydrous sodium sulfate, filtered, and the filtrate was concentrated under reduced pressure to obtain a crude product, which was purified by column chromatography (petroleum ether/ethyl acetate (V/V) = 1:0 to 0:1) to obtain 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoic acid (02C) (3.00 g, yield 87.6%).

LC-MS, M/Z (ESI): 533.5 [M-99]⁺.

### Step 3: Synthesis of benzyl 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoate (02D):

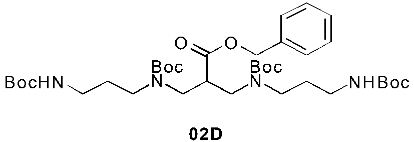

3-((Tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoic acid (3.00 g, 4.74 mmol), N,N-dimethylformamide (30 mL), potassium carbonate (1.97 g, 14.3 mmol), and benzyl bromide (0.93 g, 5.4 mmol) were sequentially added to a 500 mL single-necked flask and stirred at room temperature for 16 hours. The reaction mixture was filtered. 30 mL water was added to the filtrate, and the mixture was extracted with ethyl acetate (60 mL×3). The combined organic phase was washed with saturated brine, dried over anhydrous sodium sulfate, filtered, and the filtrate was concentrated under reduced pressure to obtain a crude product, which was purified by column chromatography (petroleum ether/ethyl acetate (V/V) = 1:0 to 1:1) to obtain benzyl 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoate (02D) (3.00 g, yield 87.5%).

¹H NMR (400 MHz, DMSO-*d*₆) δ 7.39 - 7.31 (m, 5H), 6.79 - 6.68 (m, 2H), 5.07 (s, 2H), 3.28 - 3.25 (m, 4H), 3.15 - 3.05 (m, 3H), 2.98 - 2.92 (m, 2H), 2.90 - 2.82 (m, 4H), 1.54 - 1.50 (m, 4H), 1.37 - 1.35 (m, 36H).

### Step 4: Synthesis of benzyl 3-((3-aminopropyl)amino)-2-(((3-aminopropyl)amino)methyl)propanoate tetra(trifluoroacetate) (02E):

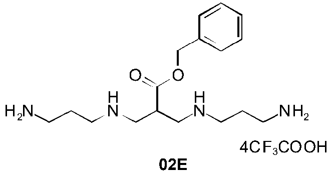

Benzyl 3-((tert-butoxycarbonyl)(3-((tert-butoxycarbonyl)amino)propyl)amino)-2-(((tert-butoxycarbonyl)(3-((tert-butoxycarbonyl)amino)propyl)amino)methyl)propanoate (4.50 g, 6.22 mmol), dichloromethane (60 mL), and trifluoroacetic acid (21.3 g, 187 mmol) were sequentially added to a 250 mL single-necked flask and stirred at room temperature for 16 hours. The reaction mixture was concentrated under reduced pressure to obtain benzyl 3-((3-aminopropyl)amino)-2-(((3-aminopropyl)amino)methyl)propanoate tetra(trifluoroacetate) (02E) (4.85 g).

LC-MS, M/Z (ESI): 323.04 [M+H]⁺.

### Step 5: Synthesis of (2R,2'R,3R,3'R,4R,4'R,5R,5'R,6R,6'R)-(((1,23-bis(((2R,3R,4R,5R,6R)-3-acetamido-4,5-diacetoxy-6-(acetoxymethyl)tetrahydro-2H-pyran-2-yl)oxy)-12-((benzyloxy)carbonyl)-5,19-dioxo-6,10,14,18-tetraazatricosane-10,14-diyl)bis(5-oxopentane-5,1-diyl))bis(oxy))bis(5-acetamido-2-(acetoxymethyl)tetrahydro-2H-pyran-6,3,4-triyl) tetraacetate (02F):

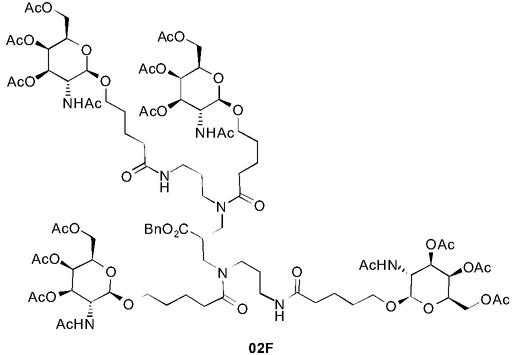

Benzyl 3-((3-aminopropyl)amino)-2-(((3-aminopropyl)amino)methyl)propanoate tetra(trifluoroacetate) (4.85 g, 6.23 mmol), dichloromethane (100 mL), 5-(((2R,3R,4R,5R,6R)-3-acetamido-4,5-diacetoxy-6-(acetoxymethyl)tetrahydro-2H-pyran-2-yl)oxy)pentanoic acid (12.55 g, 28.05 mmol), 1-ethyl-(3-dimethylaminopropyl)carbodiimide hydrochloride (7.17 g, 37.4 mmol), 1-hydroxybenzotriazole (3.37 g, 24.9 mmol), and N,N-diisopropylethylamine (12.1 g, 93.5 mmol) were sequentially added to a 250 mL single-necked flask. The mixture was stirred at room temperature for 16 hours under nitrogen protection. The reaction mixture was washed sequentially with 0.5 N aqueous hydrochloric acid (100 mL) and saturated sodium bicarbonate solution (100 mL). The combined organic phase was dried over anhydrous sodium sulfate, filtered, and the filtrate was concentrated under reduced pressure to obtain a crude product, which was purified by column chromatography (dichloromethane/methanol (V/V) = 1:0 to 9:1) to obtain (2R,2'R,3R,3'R,4R,4'R,5R,5'R,6R,6'R)-(((1,23-bis(((2R,3R,4R,5R,6R)-3-acetamido-4,5-diacetoxy-6-(acetoxymethyl)tetrahydro-2H-pyran-2-yl)oxy)-12-((benzyloxy)carbonyl)-5,19-dioxo-6,10,14,18-tetraazatricosane-10,14-diyl)bis(5-oxopentane-5,1-diyl))bis(oxy))bis(5-acetamido-2-(acetoxymethyl)tetrahydro-2H-pyran-6,3,4-triyl) tetraacetate (02F) (9.00 g, yield 70.8%).

¹H NMR (400 MHz, DMSO-*d*₆) δ 7.82 - 7.64 (m, 6H), 7.37 - 7.25 (m, 5H), 5.17 (d, *J= 3.3* Hz, 4H), 4.98 (d, *J* = 10.7 Hz, 2H), 4.93 (dd, *J=* 11.2, 3.3 Hz, 4H), 4.45 (d, *J* = 8.4 Hz, 4H), 3.98 (s, 12H), 3.84 (q, *J=* 9.2 Hz, 4H), 3.69-3.64 (m, 4H), 3.57 - 3.36 (m, 8H), 3.10 - 2.90 (m, 9H), 2.20 - 2.11 (m, 4H), 2.06 (s, 12H), 2.03 - 1.98 (m, 4H), 1.95 (s, 12H), 1.85 (s, 12H), 1.73 (s, 12H), 1.58 - 1.37 (m, 20H).

LC-MS, M/Z (ESI): 1020.9 [M+2H]²⁺.

### Step 6: Synthesis of 3-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-2-((5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)methyl)propanoic acid (02G)

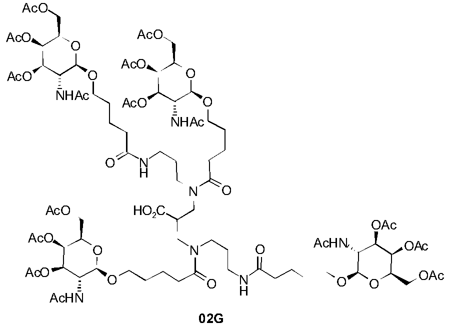

(2R,2'R,3R,3'R,4R,4'R,5R,5'R,6R,6'R)-(((1,23-bis(((2R,3R,4R,5R,6R)-3-acetamido-4,5-diacetoxy-6-(acetoxymethyl)tetrahydro-2H-pyran-2-yl)oxy)-12-((benzyloxy)carbonyl)-5,19-dioxo-6,10,14,18-tetraazatricosane-10,14-diyl)bis(5-oxopentane-5,1-diyl))bis(oxy))bis(5-acetamido-2-(acetoxymethyl)tetrahydro-2H-pyran-6,3,4-triyl) tetraacetate (0.18 g, 88 µmol), methanol (5 mL), ethyl acetate (5 mL), and 10% palladium on carbon (18 mg) were sequentially added to a 100 mL single-necked flask. The mixture was stirred at room temperature for 16 hours under hydrogen atmosphere (1 atm). The reaction mixture was filtered through diatomaceous earth. The filter cake was washed with methanol and the filtrates were combined. The filtrate was concentrated under reduced pressure to obtain 3-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-2-((5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)methyl)propanoic acid (02G) (0.12 g, yield 70%).

¹H NMR (400 MHz, DMSO-*d*₆) δ 7.83 - 7.81 (m, 5H), 7.72 - 7.68 (m, 1H), 5.21 (d, *J= 3.3* Hz, 4H), 4.96 (dd, *J* = 11.2, 3.3 Hz, 4H), 4.49 (dd, *J* = 8.4, 3.3 Hz, 4H), 4.04 - 3.99 (m, 12H), 3.87 (q, *J* = 10.5, 9.8 Hz, 4H), 3.75 - 3.66 (m, 4H), 3.59 - 3.39 (m, 8H), 3.27 - 3.13 (m, 3H), 3.08 - 2.90 (m, 6H), 2.34 - 2.15 (m, 4H), 2.10 (s, 12H), 2.07 - 2.02 (m, 4H), 1.99 (s, 12H), 1.88 (s, 12H), 1.77 (s, 12H), 1.61 - 1.43 (m, 20H).

LC-MS, M/Z (ESI): 975.9 [M+2H]²⁺.

### Step 7: Synthesis of (2R,2'R,3R,3'R,4R,4'R,5R,5'R,6R,6'R)-(((1,23-bis(((2R,3R,4R,5R,6R)-3-acetamido-4,5-diacetoxy-6-(acetoxymethyl)tetrahydro-2H-pyran-2-yl)oxy)-12-((4-nitrophenoxy)carbonyl)-5,19-dioxo-6,10,14,18-tetraazatricosane-10,14-diyl)bis(5-oxopentane-5,1-diyl))bis(oxy))bis(5-acetamido-2-(acetoxymethyl)tetrahydro-2H-pyran-6,3,4-triyl) tetraacetate (I-2):

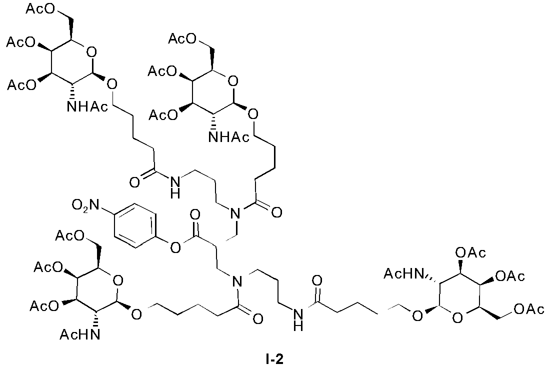

3-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-2-((5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)methyl)propanoic acid (200 mg, 0.103 mmol), dichloromethane (10 mL), 4-nitrophenol (21.4 mg, 0.154 mmol), and N,N'-dicyclohexylcarbodiimide (31.7 mg, 0.154 mmol) were sequentially added to a 100 mL single-necked flask. The mixture was stirred at room temperature for 16 hours under nitrogen atmosphere. The reaction mixture was filtered, and the filtrate was concentrated to obtain a crude product. The crude product was purified by column chromatography (dichloromethane/methanol (V/V) = 1:0 to 8:2) to obtain (2R,2'R,3R,3'R,4R,4'R,5R,5'R,6R,6'R)-(((1,23-bis(((2R,3R,4R,5R,6R)-3-acetamido-4,5-diacetoxy-6-(acetoxymethyl)tetrahydro-2H-pyran-2-yl)oxy)-12-((4-nitrophenoxy)carbonyl)-5,19-dioxo-6,10,14,18-tetraazatricosane-10,14-diyl)bis(5-oxopentane-5,1-diyl))bis(oxy))bis(5-acetamido-2-(acetoxymethyl)tetrahydro-2H-pyran-6,3,4-triyl) tetraacetate (I-2) (120 mg, yield 56.5%).

¹H NMR (400 MHz, DMSO-*d*₆) δ 8.33 - 8.29 (m, 2H), 7.79 - 7.69 (m, 6H), 7.43 - 7.31 (m, 2H), 5.18 (d, *J* = 3.3 Hz, 4H), 4.94 (dd, *J* = 11.2, 3.3 Hz, 4H), 4.45 (d, *J=* 8.4 Hz, 4H), 3.99 (s, 12H), 3.89 - 3.80 (m, 4H), 3.69 - 3.61 (m, 5H), 3.49 - 3.31 (m, 7H), 3.29 - 3.13 (m, 5H), 3.09 - 2.94 (m, 4H), 2.35 - 2.17 (m, 4H), 2.07 (d, *J* = 2.7 Hz, 12H), 2.04 - 2.01 (m, 4H), 1.96 (d, *J =* 2.0 Hz, 12H), 1.86 (s, 12H), 1.74 (s, 12H), 1.65 - 1.39 (m, 20H).

### 3. Preparation of Ligand 1-3

A synthetic route is as follows:

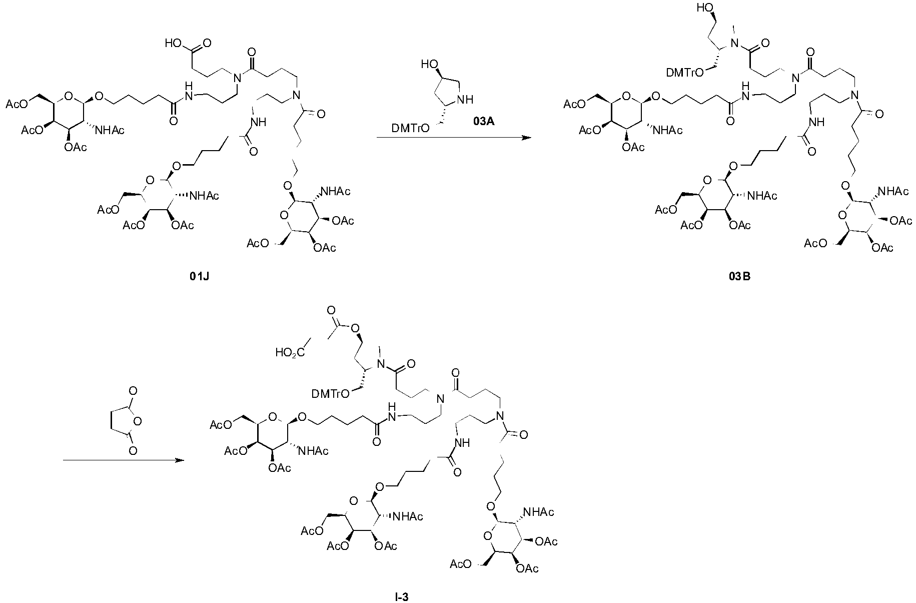

### Step 1: Synthesis of (2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-10-(4-((2S,4R)-2-((bis(4-methoxyphenyl)(phenyl)methoxy)methyl)-4-hydroxypyrrolidin-1-yl)-4-oxobutyl)-5,11,20-trioxo-6,10,15,19-tetraazaicosane-1,24-diyl)bis(oxy))bis(5-acetamido-2-(acetoxymethyl)tetrahydro-2H-pyran-6,3,4-triyl) tetraacetate (03B):

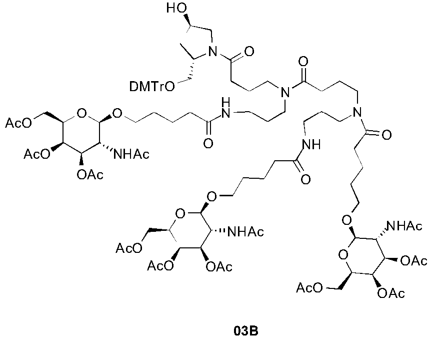

4-(4-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)butanamido)butanoic acid (01J, 1.0 g, 0.63 mmol), dichloromethane (20 mL), (3R,5S)-5-{[bis(4-methoxyphenyl)(phenyl)methoxy]methyl}pyrrolidin-3-ol (03A, 0.29 g, 0.69 mmol), benzotriazole-N,N,N',N'-tetramethyluronium hexafluorophosphate (376 mg, 0.991 mmol), 1-hydroxybenzotriazole (255 mg, 1.89 mmol), and N,N-diisopropylethylamine (244 mg, 1.89 mmol) were sequentially added to a 100 mL single-necked flask. The mixture was stirred at room temperature for 16 hours under nitrogen protection. The reaction solution was washed with saturated sodium bicarbonate solution (20 mL), dried over anhydrous sodium sulfate, filtered and concentrated to obtain a crude product. The crude product was purified by column chromatography (dichloromethane (2% triethylamine)/methanol (V/V) = 1:0 to 9:1) to obtain (2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-10-(4-((2S,4R)-2-((bis(4-methoxyphenyl)(phenyl)methoxy)methyl)-4-hydroxypyrrolidin-1-yl)-4-oxobutyl)-5,11,20-trioxo-6,10,15,19-tetraazaicosane-1,24-diyl)bis(oxy))bis(5-acetamido-2-(acetoxymethyl)tetrahydro-2H-pyran-6,3,4-triyl) tetraacetate (03B) (1.0 g, yield 80%).

¹H NMR (400 MHz, DMSO-*d*₆) δ 7.86 - 7.65 (m, 5H), 7.32 - 7.29 (m, 4H), 7.21 - 7.17 (m, 5H), 6.90 - 6.86 (m, 4H), 5.21 (s, 3H), 5.03 - 4.89 (m, 4H), 4.48 (d, *J=* 8.3 Hz, 3H), 4.36 (m, 1H), 4.16 (s, 1H), 4.02 (s, 9H), 3.87 (q, *J=* 9.8 Hz, 3H), 3.73 - 3.68 (m, 9H), 3.64 - 3.45 (m, 7H), 3.19 - 2.93 (m, 12H), 2.35 - 2.14 (m, 6H), 2.09 (s, 9H), 2.06 - 2.02 (m, 4H), 1.99 (s, 9H), 1.88 (s, 9H), 1.76 (s, 9H), 1.74 - 1.25 (m, 22H).

### Step 2: Synthesis of 4-(((3R,5S)-1-(4-(4-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)butanamido)butanoyl)-5-((bis(4-methoxyphenyl)(phenyl)methoxy)methyl)pyrrolidin-3-yl)oxy)-4-oxobutanoic acid (I-3)

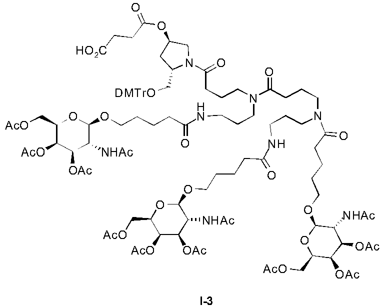

(2R,2'R,3R,3'R,4R,4'R,5R,5'R,6R,6'R)-((15-(5-(((2R,3R,4R,5R,6R)-3-acetamido-4,5-diacetoxy-6-(acetoxymethyl)tetrahydro-2H-pyran-2-yl)oxy)pentanoyl)-10-(4-((2S,4R)-2-((bis(4-methoxyphenyl)(phenyl)methoxy)methyl)-4-hydroxypyrrolidin-1-yl)-4-oxobutyl)-5,11,20-trioxo-6,10,15,19-tetraazaicosane-1,24-diyl)bis(oxy))bis(5-acetamido-2-(acetoxymethyl)tetrahydro-2H-pyran-6,3,4-triyl) tetraacetate (800 mg, 0.402 mmol), dichloromethane (30 mL), succinic anhydride (321 mg, 3.21 mmol), and 4-dimethylaminopyridine (12.3 mg, 0.101 mmol) were sequentially added to a 100 mL single-necked flask. The mixture was stirred at room temperature for 16 hours under nitrogen protection. The reaction solution was concentrated under reduced pressure. The crude product was purified by reverse-phase preparative chromatography (column: Waters Xbridge Prep C18 (150 mm×4.0 cm×10 µm); mobile phase: A = water (0.1% ammonium bicarbonate), B = acetonitrile; gradient: 15% to 45%) to obtain 4-(((3R,5S)-1-(4-(4-(5-(((2R,3R,4R,5R,6R)-3-acetamido-4,5-diacetoxy-6-(acetoxymethyl)tetrahydro-2H-pyran-2-yl)oxy)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)pentanamido)-N-(3-(5-(((2R,3R,4R,5R,6R)-3-acetamido-4,5-diacetoxy-6-(acetoxymethyl)tetrahydro-2H-pyran-2-yl)oxy)pentanamido)propyl)butanamido)butanoyl)-5-((bis(4-methoxyphenyl)(phenyl)methoxy)methyl)pyrrolidin-3-yl)oxy)-4-oxobutanoic acid (I-3) (630 mg, yield 75.0%).

¹H NMR (400 MHz, DMSO-*d*₆) δ 7.90 - 7.79 (m, 4H), 7.76 - 7.69 (m, 1H), 7.31-7.27 (m, 4H), 7.23 - 7.17 (m, 5H), 6.89 - 6.87 (m, 4H), 5.37 - 5.28 (m, 1H), 5.21 (d, *J*= 2.9 Hz, 3H), 4.97 (dd, *J* = 11.2, 3.0 Hz, 3H), 4.49 (d, *J* = 8.4 Hz, 3H), 4.21 (s, 1H), 4.02 (s, 9H), 3.91 - 3.84 (m, 3H), 3.73 - 3.70 (m, 9H), 3.56 - 3.39(m, 7H), 3.23 - 3.19 (m, 8H), 3.04 - 2.97 (m, 4H), 2.49 - 2.40 (m, 4H), 2.29 - 2.17 (m, 6H), 2.09 (s, 9H), 2.05 - 2.04 (m, 4H), 1.99 (s, 9H), 1.89 (s, 9H), 1.77 (s, 9H), 1.72 - 1.40 (m, 22H).

### Example 1: Design and Synthesis of siRNA or Conjugates Thereof

### 1. Design and Synthesis of siRNA Conjugates

1.1 Synthesis of siRNA Sequences in Which the Conjugation Group Is Linked to the 3'-End of the Sense Strand of siRNA. Taking C3 in Table 1 as an example, the specific steps are as follows:
1) Preparation of GalNAc Solid Support: Ligand I-3 prepared in Preparation Example 1 was condensed with aminomethyl resin (CPG, DinaXingke) under HBTU/DIEA conditions to obtain I-3-CPG. The loading capacity of the prepared support was determined and used for subsequent solid-phase synthesis.
2) Synthesis of Single-Stranded Oligoribonucleotides: Oligoribonucleotides were synthesized by phosphoramidite solid-phase synthesis on the prepared I-3-CPG (500 Å). All 2'-modified RNA phosphoramidite monomers (Tangzhi Pharmaceutical) and auxiliary reagents were commercially available reagents. All phosphoramidites were dissolved in anhydrous acetonitrile (Kelema, Suzhou) and molecular sieves (3 Å) were added. 5-Ethylthio-1H-tetrazole (ETT) (Kelema, Suzhou) was used as the activator with a coupling time of 8 minutes to 12 minutes. A 0.05 M iodine solution (dissolved in pyridine/water = 9:1, Kelema, Suzhou) was used to form phosphate linkage. A solution of 0.2 M xanthane hydride (Kelema, Suzhou) in anhydrous acetonitrile/pyridine (v/v = 1/1) was used to generate phosphorothioate linkage with a reaction time of 5 minutes. Synthesis was completed after final removal of DMT groups.
3) Cleavage and Deprotection of Oligoribonucleotides from CPG: After completion of solid-phase synthesis, protecting groups were removed by treatment with acetonitrile containing 20% diethylamine (Sinopharm) for 10 minutes. The resulting CPG support was then treated with concentrated aqueous ammonia (Sinopharm) under heating for ammonolysis to remove the protecting groups on the support and bases. After filtration, a solution containing the product was obtained.
4) Purification of Single-Stranded Oligoribonucleotides: Oligomers were obtained by HPLC purification using NanoQ anion exchange. Buffer A was a 20 mM sodium hydroxide solution and Buffer B contained a 20 mM sodium hydroxide solution and 3 M sodium chloride. The target product was isolated and subsequently desalted by gel filtration.
5) The chemically synthesized sense strand and antisense strand were mixed at a molar ratio of 1:1 for complementary base pairing. The reaction was carried out at 70°C for 10 minutes and then slowly returned to room temperature to obtain the final product.

1.2. Synthesis of siRNA Conjugates in Which the Conjugating Group Is Linked to the 5' End of the Sense Strand (i.e., C1-C2, C4-C5). Taking C1 in Table 1 as an example, the specific steps are as follows:
1) Synthesis of Single-Stranded Oligoribonucleotides: Oligoribonucleotides were synthesized by phosphoramidite solid-phase synthesis on universal controlled pore glass CPG (500 Å). All phosphoramidite monomers (Tangzhi Pharmaceutical & Shanghai Zhaowei) and auxiliary reagents were commercially available reagents. Phosphoramidites containing the conjugation group were prepared from the corresponding compounds in Example 1. All phosphoramidites were dissolved in anhydrous acetonitrile (Kelema, Suzhou) and molecular sieves (3 Å) were added. 5-Ethylthio-1H-tetrazole (ETT) (Kelema, Suzhou) was used as the activator with a coupling time of 8 minutes to 12 minutes. A 0.05 M iodine solution (dissolved in pyridine/water = 9:1, Kelema, Suzhou) was used to form phosphate linkage. A solution of 0.2 M xanthane hydride (Kelema, Suzhou) in anhydrous acetonitrile/pyridine (v/v = 1/1) was used to generate phosphorothioate linkage with a reaction time of 5 minutes. Synthesis was completed after final removal of DMT groups.
2) Cleavage and Deprotection of Oligoribonucleotides from CPG: After completion of solid-phase synthesis, protecting groups were removed by treatment with acetonitrile containing 20% diethylamine (Sinopharm) for 10 minutes. The resulting CPG support was then treated with concentrated aqueous ammonia (Sinopharm) under heating for ammonolysis to remove the protecting groups on the support and bases. After filtration, a solution containing the product was obtained.
3) Purification of Single-Stranded Oligoribonucleotides: Oligomers were obtained by HPLC purification using NanoQ anion exchange. Buffer A was a 20 mM sodium hydroxide solution and Buffer B contained a 20 mM sodium hydroxide solution and 3 M sodium chloride. The target product was isolated and subsequently desalted by gel filtration.
4) Conjugation and Purification of Conjugation Group: A purified nucleic acid sequence with a terminal NH₂-C₆ group (prepared and purified by conventional methods in the art) was first dissolved in 100 mM phosphate buffer (PB). Ligand I-1 or I-2 in Preparation Example 1 was dissolved in DMF or DMSO and then added to the nucleic acid solution. The reaction was carried out at room temperature for 4 hours to 16 hours. Completion of the reaction was monitored by mass spectrometry. Then, AMA solution (25% aqueous ammonia: 40% methylamine aqueous solution = 1:1) in twice the volume of the reaction mixture was added and stirred at room temperature for 0.5 hours, followed by quenching with water. The mixed solution was desalted to remove organic reagents followed by purification to obtain a nucleic acid strand with a terminal conjugation group.
5) The chemically synthesized sense strand and antisense strand were mixed at a molar ratio of 1:1 for complementary base pairing. The reaction was carried out at 70°C for 10 minutes and then slowly returned to room temperature to obtain the final product.

The nucleotide sequences of the sense strand and the antisense strand of the siRNA conjugates obtained in the present disclosure are shown in Table 1. The specific structures of ligands (II-1, II-2, II-3) in Table 1 are described in the Summary of this specification (i.e., II-1, II-2, II-3, the compound or the stereoisomer, tautomer or pharmaceutically acceptable salt thereof according to the third aspect). II-1 is derived from ligand I-1, II-2 is derived from ligand 1-2, and II-3 is derived from ligand I-3.

**[Table 1]:**

| Group | Nucleotide sequence contained in sense strand (5'-3') | Nucleotide sequence contained in antisense strand (5'-3') |
|---|---|---|
| AD-56001 8 | asasgagaAfgUfCfGfuuucauucau(L96) | asUfsgaaUfgAfAfacgaCfuUfcucuusgsu |
| C1 | (II-1)s(invAb)saagagaagUfcGfuUfucauucaus(invAb) | asUfsgaauGfaaacGfaCfuUfcucsusu |
| C2 | (II-2)s(invAb)saagagaagUfcGfuUfucauucaus(invAb) | asUfsgaauGfaaacGfaCfuUfcucsusu |
| C3 | asasgagaAfgUfCfGfuuucauucau(II-3) | asUfsgaaUfgAfAfacgaCfuUfcucuusgsu |
| C4 | (II-1)s(invAb)suauggaaaAfcCfuGfgaagaugus(invAb) | EVpasCfsaucuUfccagGfuUfuUfccasusa |
| C5 | (II-2)s(invAb)suauggaaaAfcCfuGfgaagaugus(invAb) | EVpasCfsaucuUfccagGfuUfuUfccasusa |

Preparation of AD-560018 is described in Patent WO2021/222549A1. In Table 1, lowercase letters "c", "g", "u", "a", and t each represent a 2'-methoxy-modified nucleotide (i.e., "c", "g", "u", and "a" each indicate that 2'-OH of the ribosyl group of the nucleotide represented by the corresponding uppercase letter is substituted with a methoxy group); "f" indicates that the nucleotide represented by a preceding letter is a 2'-fluoro-modified nucleotide (i.e., 2'-OH of the nucleotide represented by a letter preceding "f' is substituted with a fluorine atom); and "s" indicates that the two adjacent nucleotides flanking "s" are connected via a phosphorothioate linkage (i.e., the 5'-phosphate group of the nucleotide represented by a letter preceding "s" is substituted with a 5'-phosphorothioate group).

At present, five marketed GalNAc-siRNA conjugates (Givosiran, Inclisiran, Lumasiran, Vutrisiran, etc.) use L96 as the ligand scaffold. The preparation method thereof is described in WO2014/025805.

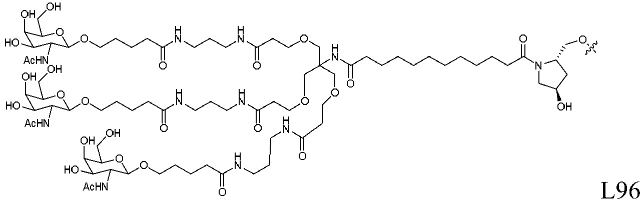

The structures of EVpu and EVpa are shown below:

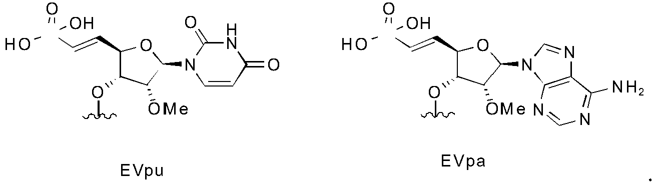

### 2. Unmodified siRNA sequences corresponding to C4 in Table 1 (abbreviated as siRNA-4) was synthesized by the following method:

The oligoribonucleotide was synthesized by phosphoramidite solid-phase synthesis on universal controlled pore glass CPG (500 Å) or ligand-coupled CPG. All 2'-modified RNA, DNA, phosphoramidites of ligands, and auxiliary reagents were commercially available reagents (Tangzhi Pharmaceutical). All phosphoramidites were dissolved in anhydrous acetonitrile and molecular sieves (3 Å) were added. 5-Ethylthio-1H-tetrazole (ETT, Suzhou Kelema Biotechnology Co., Ltd.) was used as the activator with a coupling time of 200 seconds, and ligand coupling time was 10 minutes. 50 nM iodine in pyridine (Sinopharm)/water was used to form phosphate linkage with a reaction time of 5 minutes. 0.2 M xanthane hydride (Shanghai Zhaowei Technology Development Co., Ltd.) in pyridine (Sinopharm) was used to generate phosphorothioate linkage with a reaction time of 3 minutes. Synthesis was completed after final removal of DMT groups.

After completion of solid-phase synthesis, the dried solid support was treated with aqueous ammonia at 55°C for 16 hours. Certain monomers were deprotected using a DMSO/triethylamine trihydrofluoride (Beijing J&K Scientific Ltd.) mixture (5:1) at 25°C for 4 hours. The solution was evaporated and the solid residue was redissolved in water.

The crude product was purified by reversed-phase HPLC using a Waters XBridge C18 column and an Autotide 100 system. Buffer A was 100 mM TEAA with a pH value of 7.5 and containing 5% acetonitrile, and Buffer B was 100% acetonitrile. UV traces were recorded at 260 nm and appropriate fractions were collected.

For annealing, single-stranded oligoribonucleotides were prepared as 200 µM solutions in sterile nuclease-free water. The annealing reaction system was set as follows: a mixture with a total volume of 100 µL containing 10 nmol was placed in a 95°C water bath for 5 minutes (≥100 nmol requires 20 minutes), then immediately placed into a 60°C water bath and allowed to cool naturally. The solution after annealing shall not be stored at high temperature. Complementary strands were formed by combining equimolar single-stranded oligoribonucleotide solutions to finally obtain siRNA-4. The molecular weight of siRNA was measured by liquid chromatography-mass spectrometry (LC-MS) to compare the measured molecular weight with the theoretical value. Results showed that the measured value was approximately equal to the theoretical value, confirming that siRNA-4 was obtained. The specific nucleotide sequences are: UAUGGAAAACCUGGAAGAUGU (sense strand); ACAUCUUCCAGGUUUUCCAUAUU (antisense strand).

### Test Example 1: Inhibition of CFB mRNA Expression in Primary Human Hepatocytes by Free Uptake of siRNA Conjugates

The siRNA conjugates prepared in Example 1 were prepared into stock solutions at the corresponding concentrations with Nuclease-Free Water.

The diluted test siRNA conjugates were added to collagen-coated 96-well cell plates, followed by addition of primary human hepatocyte (PHH) (from WuXi AppTec Chengdu) as a cell suspension. A control group containing Nuclease-Free Water without siRNA conjugates was set simultaneously. The cells were cultured in an incubator at 37°C with 5% CO₂ for 48 hours.

After 48 hours of free uptake, the culture medium was removed, and the cell plates were washed once with PBS. Cell lysis buffer was added. Total RNA was extracted using an RNA extraction kit (Qiagen-74182). gDNA was removed using a reverse transcription kit HiScript III RT SuperMix for qPCR (Vazyme-R323-01), and cDNA was synthesized by reverse transcription with random primers.

The cDNA of the target gene was detected by qPCR, and the corresponding internal reference gene (GAPDH cDNA) was detected in parallel. The qPCR reaction program (TaqMan Probe) was as follows: 50°C for 2 minutes, heating at 95°C for 10 minutes; followed by 40 cycles of heating at 95°C for 10 seconds and 60°C for 1 minute.

The mRNA expression level of the target gene in each sample was calculated by a ΔΔCT quantitative method. The relative expression level of the target gene was presented as 2-ΔΔCT.

The calculation formulas are as follows:
$ΔCT = average Ct value of target gene - average Ct value of internal reference gene ;$
$ΔΔCT = ΔCT \left(treatment group\right) - ΔCT \left(Nuclease-Free Water control group\right) ;$
$Relative expression level of target gene = 2 - ΔΔCT ;$ 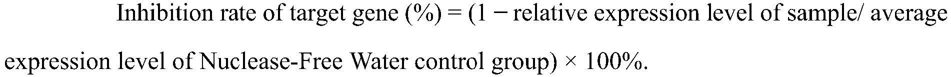 Inhibition rate of target gene (%) = (1 - relative expression level of sample/ average expression level of Nuclease-Free Water control group) × 100%.

The inhibition rate is presented as mean + SD. The test results are shown in Table 2.

**[Table 2]**

| **siRNA Conjugate Group (corresponding to Table 1)** | **Absolute EC₅₀ (nM)** |
|---|---|
| C1 | 0.125 |
| C2 | 0.145 |

The results indicate that the siRNA conjugates of the present disclosure exhibit a significant silencing effect on CFB gene expression in primary human hepatocytes under free uptake conditions. It can be concluded that the targeting ligands of the present disclosure can significantly improve the ability of the siRNA conjugates to enter liver cells, without obvious impact on the silencing effect of siRNA.

### Test Example 2: Inhibition of CFB mRNA Expression in Hep3B Cells by siRNA and Conjugates Thereof

siRNA conjugate: prepared into 20 µM stock solutions with Nuclease-Free Water.

Hep3B cells were provided by WuXi AppTec Co., Ltd (Chengdu). Hep3B cells were cultured in EMEM medium containing 10% fetal bovine serum, 1% glutamine, 1% NEAA, and 1% penicillin-streptomycin.

The primer/probe set for target gene CFB (Thermo, Assay ID: Hs00156060_m1) was purchased from Thermo Fisher Scientific Inc. Taqman primer/probe set for internal reference gene GAPDH were designed and provided by WuXi.

### Experimental Method:

Hep3B cells were seeded into 96-well cell plates (2×10⁴ cells per well). At the time of seeding, siRNA was transfected into cells using Lipofectamine^{™} RNAiMAX. Nine concentration points of siRNA were tested (starting from 10 nM, with 6-fold dilutions), with 3 replicate wells. Also, a control group containing Lipofectamin^{™} RNAiMAX (without compound) was set. The cells were cultured in an incubator at 37°C with 5% CO₂ for 48 hours.

After 48 hours of transfection, the culture medium was removed and the cells were harvested for RNA extraction. Total RNA was extracted using the RNeasy^{®} 96 Kit (QIAGEN-74182) based on kit instructions. cDNA was synthesized using the FastKing RT kit (with gDNase)/FastKing First Strand cDNA Synthesis Kit (TIANGEN-KR116-03) based on the instructions.

Target gene cDNA was detected by qPCR, and GAPDH cDNA was detected in parallel as an internal control. In a 384-well plate, 8 µL of prepared PCR reaction solution and 2 µL of sample cDNA were added. The qPCR reaction program was as follows: preheating at 50°C for 2 minutes, heating at 95°C for 10 minutes; followed by 40 cycles of heating at 95°C for 10 seconds and 60°C for 1 minute.

The mRNA expression level of the target gene in each sample was calculated by a ΔΔCT relative quantitative method. The relative expression level of the target gene was presented as 2-ΔΔCT.

The calculation formulas are as follows:
$ΔCT = average Ct value of target gene - average Ct value of internal reference gene ;$
$ΔΔCT = ΔCT \left(sample group\right) - ΔCT \left({Lipofectamine}^{TM} RNAiMAX control group\right) ;$
$Relative expression level of target gene = 2 - ΔΔCT ;$ 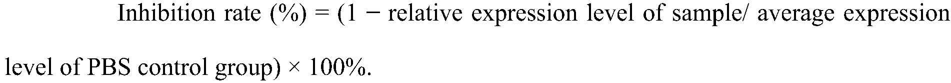
Inhibition rate (%) = (1 - relative expression level of sample/ average expression level of PBS control group) × 100%.

The test results are shown in Table 3.

**[Table 3]**

| **siRNA Conjugate Group (corresponding to Table C)** | **Absolute EC₅₀ (pM)** | **Maximum inhibition rate (%)** |
|---|---|---|
| siRNA-4 | 12 | 90.7 |
| C4 | 26 | 92.4 |

The results indicate that the siRNA conjugates of the present disclosure exhibit a significant silencing effect on CFB gene expression in Hep3B cells. It can be concluded that the targeting ligands of the present disclosure can significantly improve the ability of the siRNA conjugates to enter Hep3B cells, without obvious impact on the silencing effect of siRNA.

### Test Example 3: Inhibition of hCFB Expression in Tg-hCFB Mice by siRNA Conjugates

Six- to eight-week-old male Tg-hCFB transgenic mice (purchased from Biocytogen Jiangsu Gene Biotechnology Co., Ltd.) were used to evaluate the in vivo efficacy of CFB siRNA conjugates.

The test compounds (siRNA conjugates obtained in Example 1) were prepared using physiological saline (0.4 mg/mL) on the day of administration, stored at room temperature, and administered within 2 hours. The remaining test compounds were stored in a -20 °C refrigerator. Animals were divided into a normal control group and four treatment groups, with 5 mice per group. Grouping was performed based on an hCFB protein level in mouse serum (blood collected on Day-3). The treatment groups received a single subcutaneous injection of the test compound, and the normal control group received an equivalent volume of physiological saline. The day of administration was recorded as Day 0. Body weight was recorded once a week starting from one day before administration.

Approximately 200 µL of blood was collected from the medial canthus of each mouse on the day before administration (Day-1), and on Day 7, Day 14, Day 21, Day 28, and Day 35 after administration. Whole blood samples were placed in a 37°C water bath for 1 hour before centrifugation, and then centrifuged at 3000 rpm for 10 minutes. Supernatant was collected as a freshly prepared serum sample, and hCFB protein was detected by an ELISA method. At the experimental endpoint, livers of the mice were harvested, and hCFB mRNA expression in liver tissue was detected by qPCR.

Experimental data are expressed as mean ± standard deviation (Mean ± SD) and were analyzed and plotted using GraphPad Prism 8.3 software. Statistical analysis was performed using a t-test, with P < 0.05 considered statistically significant. For each animal, the hCFB level at each time point was divided by the corresponding pre-administration level (Day -1) to determine the expression remaining rate. The individual expression remaining rate in the treatment group at a certain time point was divided by the average expression remaining rate of all mice in the control group to obtain a "normalized remaining expression rate", and the average value of each group was calculated. The test results are shown in Table 4 and FIG. 1.

**[Table 4]**

| siRNA Conjugate | Serum Normalized Expression Remaining Rate (%) | | | | | | | hCFB mRNA remaining rate (%) |
|---|---|---|---|---|---|---|---|---|
| | Day 3 | Day 7 | Day 14 | Day 21 | Day 28 | Day 35 | Day 42 | |
| Control group | 101.1±7. 39 | 103.5±2 8.1 | 104.9±3 7.13 | 101.8±2 0.5 | 102.2±2 4.63 | 101.3±1 7.48 | 101.4±2 1.98 | 110+55 |
| AD-560018, 2 mpk | 22.3+5.8 7 | 7.8±1.35 | 4.5±3.35 | 11.3±2.5 4 | 10+3.47 | 22±4.71 | 31.8±9.9 5 | 80+31 |
| C1, 2 mpk | 26.3±4.4 6 | 5.8±1.8 | 4.6+2.51 | 7.7±1.06 | 5.4+0.91 | 8.6±4.97 | 8.4±3.77 | 70±35 |
| C2, 2 mpk | 24.7±4.2 6 | 8.7±1.03 | 6.4+5.17 | 8.9±2.48 | 5.9±1.55 | 12±1.61 | 11.7±2.9 4 | 50+28 |
| C3, 2 mpk | 23.8±6.6 1 | 5.3±1.72 | 7.7±3.1 | 14.9+3.4 8 | 12.3±4.2 2 | 28.2±6.1 | 41.2±13. 5 | 40±7 |

The results indicate that the siRNA conjugates of the present disclosure exhibit a significant and sustained silencing effect on hCFB expression in Tg-hCFB mice, indicating that the ligands provided in the present disclosure demonstrate superior liver-targeted delivery effects compared to L96.

### Test Example 4: In Vitro Liver S9 Stability Experiment of siRNA Conjugates

Test compound stock solution: 2 mg/mL solution in nuclease-free water.

Reference compound stock solution: 2 mM solution in nuclease-free water; diluted to 20 µM with nuclease-free water before use.

Seven 96-well incubation plates were prepared and designated as BK, T1440, T360, T240, T120, T60, and T0 incubation plates, respectively. 190 µL of liver S9 working solution (liver S9 protein concentration: 1.05 mg/mL) was added to each incubation plate (BK, T1440, T360, T240, T120, T60, and T0). Subsequently, 200 µL of stop solution was added to the T0 termination plate, and the stop solution was 30% ammonia aqueous solution in nuclease-free water containing 2.50 mM TCEP (tris(2-carboxyethyl)phosphine, Thermo, ZG394848) and 2000 ng/mL IS-WuXi.

Except for the BK plate, 10 µL of compound was added to each well of the incubation plates (T1440, T360, T240, T120, T60, and T0) to initiate a reaction. Therefore, in the samples of the test compound or the reference compound (Inclisiran), the final reaction concentration of the test compound or the reference compound was 1 µM, and the concentration of human liver S9 (BioIVT) was 1.0 mg/mL.

After incubation at 37°C for appropriate time periods (60, 120, 240, 360, and 1440 minutes), 200 µL of stop solution (30% ammonia aqueous solution in nuclease-free water containing 2.50 mM TCEP and 2000 ng/mL IS-WuXi) was added to the sample well of each termination plate, and the plates were sealed.

All sample plates were shaken for 10 minutes. Then, 200 µL of DNA extraction reagent and 500 µL of dichloromethane were added to each sample well and mixed thoroughly by shaking. All sample plates were shaken and centrifuged at 3220×g for 20 minutes at 4°C, and then 100 µL of supernatant was collected from each well for liquid chromatography-tandem mass spectrometry (LC-MS/MS) analysis.

In this test example, samples were analyzed by LC-MS/MS. According to the following formula, the percentage remaining was calculated based on the peak area ratio of the compound to the internal standard (IS-WuXi), to determine the in vitro elimination rate constant kₑ of the test compound and the reference compound accordingly: $% Remaining = \frac{Peak area ratio \left(compound/IS\right) at time t}{Peak area ratio \left(compound/IS\right) at time 0} \times 100$ ${C}_{t} = {C}_{0} ⋅ {e}^{- {k}_{{e}^{. t}}}$ when ${C}_{t} = \frac{1}{2} {C}_{0}$, ${T}_{1 / 2} = \frac{Ln 2}{{k}_{e}} = \frac{0.693}{{k}_{e}}$

If the percentage remaining of a compound at the maximum reaction time point (1440 minutes in this experiment) was greater than 75%, the compound was considered metabolically stable within the acceptable experimental variability (CV = 25%). That is, the corresponding critical half-life value was defined as T_{1/2} > 3469 minutes. Based on the above formula, the intrinsic clearance in human liver S9 (CL_{int(S9)}) was calculated to be < 0.2 µL/min/mg protein. The experimental results are shown in Table 5, where AS and SS in the sample names represent the antisense strand and the sense strand of the siRNA conjugate, respectively.

**[Table 5]**

| Sample name | Human liver S9 | | |
|---|---|---|---|
| | T_{1/2} (minutes) | CL**_{int(S9)}** (µL/min/mg) | Remaining rate |
| AD-560018-AS | >3469 | <0.2 | 81.1% |
| AD-560018-SS | 467.2 | 1.48 | 14.0% |
| C1-AS | >3469 | <0.2 | 91.9% |
| C1-SS | 656.9 | 1.06 | 20.2% |
| C2-AS | >3469 | <0.2 | 97.3% |
| C2-SS | 584.3 | 1.19 | 19.2% |

The results indicate that the sense strand of the siRNA conjugates of the present disclosure exhibits a higher remaining rate in human liver S9, and the antisense strand exhibits a higher remaining rate and a lower intrinsic clearance in human liver S9. For siRNA conjugates having identical sequences, the targeting ligands provided in the present disclosure demonstrate superior human metabolic stability compared with L96 used in AD-560018.

### Test Example 5: In Vitro Oligonucleotide Liver Lysosome Stability Experiment of siRNA Conjugates

Test compound stock solution: 2 mg/mL solution in nuclease-free water.

Reference compound stock solution: 2 mM solution in nuclease-free water; diluted to 20 µM with nuclease-free water before use.

Seven 96-well incubation plates were prepared and designated as BK, T1440, T360, T240, T120, T60, and T0 incubation plates, respectively. 190 µL of human liver lysosome working solution (liver lysosome protein concentration: 1.05 mg/mL) was added to each incubation plate (BK, T1440, T360, T240, T120, T60, and T0), respectively. Subsequently, 200 µL of stop solution (30% ammonia aqueous solution in nuclease-free water containing 2.50 mM TCEP and 2000 ng/mL IS-WuXi) was added to the T0 termination plate.

Except for the BK plate, 10 µL of compound was added to each well of the incubation plates (T1440, T360, T240, T120, T60, and T0) to initiate a reaction. Therefore, in the samples of the test compound or the reference compound (Inclisiran), the final reaction concentration of the test compound and the reference compound was 1 µM, and the concentration of human liver lysosome was 1.0 mg/mL.

After incubation at 37°C for appropriate time periods (60, 120, 240, 360, and 1440 minutes), 200 µL of stop solution (30% ammonia aqueous solution in nuclease-free water containing 2.50 mM TCEP and 2000 ng/mL IS-WuXi) was added to the sample well of each termination plate, and the plates were sealed.

All sample plates were shaken for 10 minutes. Then, 200 µL of DNA extraction reagent and 500 µL of dichloromethane were added to each sample well and mixed thoroughly by shaking. All sample plates were shaken and centrifuged at 3220×g for 20 minutes at 4°C, and then 100 µL of supernatant was collected from each well for liquid chromatography-tandem mass spectrometry (LC-MS/MS) analysis.

In this test example, samples were analyzed by LC-MS/MS for the test compound and the reference compound. According to the following formula, the percentage remaining was calculated based on the peak area ratio of the compound to the internal standard (IS-WuXi), to determine the in vitro elimination rate constant kₑ of the test compound and the reference compound accordingly: $% Remaining = \frac{Peak area ratio \left(compound/IS\right) at time t}{Peak area ratio \left(compound/IS\right) at time 0} \times 100$ ${C}_{t} = {C}_{0} ⋅ {e}^{- {k}_{{e}^{. t}}}$ when ${C}_{t} = \frac{1}{2} {C}_{0}$, ${T}_{1 / 2} = \frac{Ln 2}{{k}_{e}} = \frac{0.693}{{k}_{e}}$

If the percentage remaining of a compound at the maximum reaction time point (1440 minutes in this experiment) was greater than 75%, the compound was considered metabolically stable within the acceptable experimental variability (CV = 25%). That is, the corresponding critical half-life value was defined as T_{1/2} > 3469 minutes. Based on the above formula, the intrinsic clearance in liver lysosome (CL_{int(LYS)}) was calculated to be < 0.2 µL/min/mg protein. The experimental results are shown in Table 6, where AS and SS in the sample names represent the antisense strand and the sense strand of the siRNA conjugate, respectively.

**[Table 6]**

| Sample name | Human liver lysosome | | |
|---|---|---|---|
| | T_{1/2} (minutes) | CL_{int(LYS)} (µL/min/mg) | Remaining rate |
| AD-560018-AS | >3469 | <0.2 | 82.2% |
| AD-560018-SS | 1022.6 | 0.68 | 27.8% |
| C₁₋AS | >3469 | <0.2 | 88.3% |
| C₁₋SS | 1073.8 | 0.65 | 30.6% |
| C2-AS | >3469 | <0.2 | 94.0% |
| C2-SS | 1235.5 | 0.56 | 31.8% |

The results indicate that the sense strand of the siRNA conjugates of the present disclosure exhibits a higher remaining rate in human liver lysosome, and the antisense strand exhibits a higher remaining rate and a lower intrinsic clearance in human liver lysosome. For siRNA conjugates having identical sequences, the targeting ligands provided in the present disclosure demonstrate superior human metabolic stability compared with L96 used in AD-560018.

### Test Example 6: Toxicity Evaluation Experiment of siRNA Conjugates in Rats

Sprague-Dawley (SD) rats (purchased from Beijing Vital River Laboratory Animal Technology Co., Ltd.), SPF grade, 6 to 9 weeks old, body weight 210 to 250 g, were randomly grouped based on body weight. Subcutaneous administration was performed once every two weeks (Day 1, Day 14, and Day 28). The specific dose design and grouping are detailed as follows:

| Group Code | Group Name | Dose (mg/kg) | Number of Animals | |
|---|---|---|---|---|
| | | | Female | Male |
| G1 | Vehicle control group | 0 | 3 | 3 |
| G2 | AD-560018-low-dose group | 30 | 3 | 3 |
| G3 | AD-560018-high-dose group | 100 | 3 | 3 |
| G4 | C1-low-dose group | 30 | 3 | 3 |
| G5 | C1-high-dose group | 100 | 3 | 3 |
| G6 | C2-low-dose group | 30 | 3 | 3 |
| G7 | C2-high dose group | 100 | 3 | 3 |

### Test indices:

Clinical observation: Clinical observation was performed twice per day.

Body weight: Body weights of all surviving animals were measured twice per week.

Food consumption: Food consumption of all surviving animals was measured twice per week.

Hematology and serum biochemistry: Blood samples were collected on Day 29 after administration for analyses of hematology and serum biochemistry.

Gross necropsy and histopathological examination: After blood collection, the animals were dissected, and major organs were collected. Livers, spleens, and kidneys were weighed and subjected to histopathological examination. The test results are as follows:

| Group Code | ALT (alanine aminotransferase (U/L) | | AST (aspartate aminotransferase) (U/L) | | ALP (alkaline phosphatase) (U/L) | |
|---|---|---|---|---|---|---|
| | Male | Female | Male | Female | Male | Female |
| G1 | 53.18±8.17 | 46.78±1.76 | 211.37±60.95 | 116.50±2.06 | 184.71+35.04 | 97.84+0.59 |
| G2 | 58.07±4.11 | 54.51±10.21 | 189.29±11.42 | 210.99+87.28 | 230.75±16.12 | 163.54+61.4 |
| G3 | 68.72±10.85 | 46.73±12.07 | 208.61±9.39 | 168.94±18.07 | 553.08±33.9 | 329.38+38.59 |
| G4 | 57.35±10.29 | 42.26±9.15 | 145.29±18.20 | 143.23±18.48 | 227.81±33.41 | 112.81+30.76 |
| G5 | 59.98±7.32 | 40.43±10.35 | 200.23±45.68 | 108.67±11.34 | 254.57±20.35 | 118.57+22.26 |
| G6 | / | 35.62+2.69 | 187.07±22.29 | 112.4149.31 | 214.85±13.63 | 99.77+53.81 |
| G7 | 63.68±19.39 | 40.39+8.41 | 175.16+38.7 | 126.86±17.73 | 287.78±7.47 | 118.26+21.64 |

The results indicate that, compared with AD-560018, the siRNA conjugates prepared using the ligands of the present disclosure induce less elevation of major liver function indicators, including ALT, AST, and ALP. The siRNA conjugates C1 and C2 provided in the present disclosure exhibit lower liver toxicity and an excellent therapeutic safety window. It can be demonstrated that for siRNA conjugates having identical sequences, the targeting ligands provided in the present disclosure have superior safety compared with L96.

Reference throughout this specification to "an embodiment", "some embodiments", "an example", "a specific example", or "some examples" means that a particular feature, structure, material, or characteristic described in connection with the embodiment or example is included in at least one embodiment or example of the present disclosure. In this specification, exemplary descriptions of above terms are not necessarily referring to the same embodiment or example. Further, the particular features, structures, materials, or characteristics can be combined in any suitable manner in one or more embodiments or examples. In addition, different embodiments or examples and the features of different embodiments or examples described in the specification may be combined and integrated by those skilled in the art without mutual contradiction.

Although embodiments of the present disclosure have been shown and described above, it should be understood that above embodiments are merely exemplary, and cannot be construed to limit the present disclosure. For those skilled in the art, various changes, modifications, replacements, and variations can be made to the embodiments without departing from the scope of the present disclosure.

## Claims

1. A compound represented by formula (I), or a stereoisomer, tautomer or pharmaceutically acceptable salt thereof: 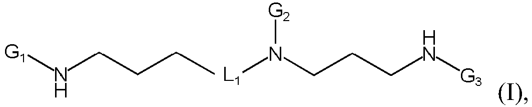 wherein:
L₁ is -N(R₁)-C(O)-(C₁₋₁₀ alkylene)- or -N(G₄)-L₂-;
L₂ is C₁₋₆ alkylene substituted with R₁;
G₁, G₂, G₃, and G₄ are each independently 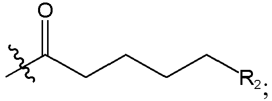
R₂ is a residue of a substituted or unsubstituted saccharide;
R₁ is -(C₀₋₁₀ alkylene)-C(O)-R₁₀; and
R₁₀ is a leaving group.

2. The compound represented by formula (I), or the stereoisomer, tautomer or pharmaceutically acceptable salt thereof according to claim 1, satisfying at least one of the following conditions:
1) L₂ is C₂₋₄ alkylene substituted with R₁;
2) L₁ is -N(R₁)-C(O)-(C₁₋₆ alkylene)- or -N(G₄)-CH₂-CH(R₁)-CH₂-;
3) R₁₀ is selected from 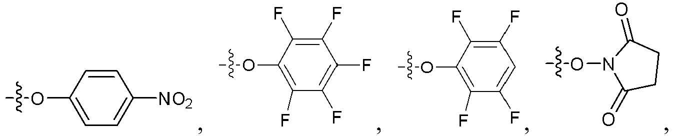 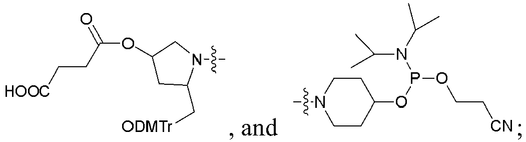 preferably, 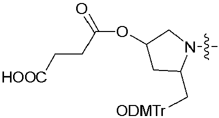 is 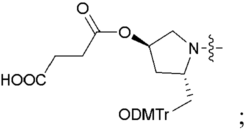
4) R₁ is selected from 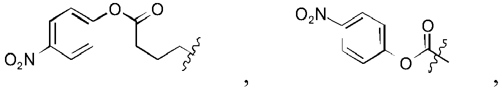 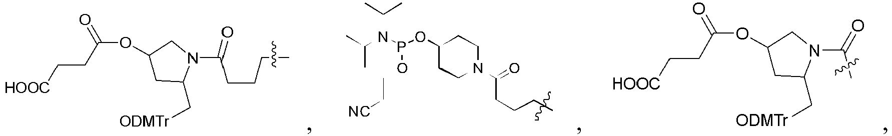 and 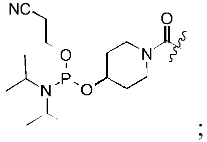
preferably, 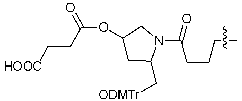 is 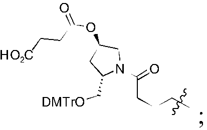
preferably, 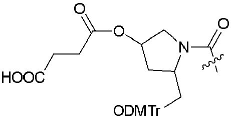 is 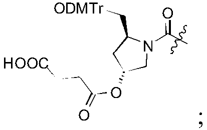
5) the saccharide is glucose, lactose, galactose, or fructose;
6) R₂ is independently a residue of a compound selected from N-acetylgalactosamine, N-trifluoroacetylgalactosamine, N-formylgalactosamine, N-propionylgalactosamine, N-n-butyrylgalactosamine, and N-isobutyrylgalactosamine; or
7) G₁, G₂, G₃, and G₄ are each independently 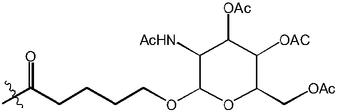 or 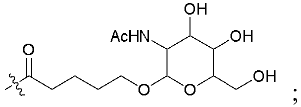 preferably, G₁, G₂, G₃, and G₄ are each independently 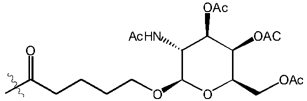 or

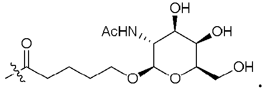

3. The compound represented by formula (I), or the stereoisomer, tautomer or pharmaceutically acceptable salt thereof according to claim 1 or 2, wherein the compound represented by formula (I) has a structure represented by formula (Ia) or formula (Ib): 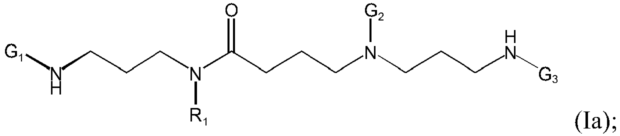 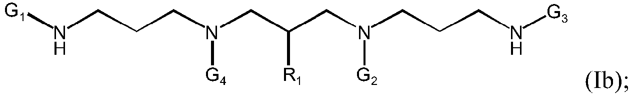 wherein:
in formula (Ia), R₁ is -(C₁₋₆ alkylene)-C(O)-R₁₀; and
in formula (Ib), R₁ is -C(O)-R₁₀.

4. The compound represented by formula (I), or the stereoisomer, tautomer or pharmaceutically acceptable salt thereof according to claim 1, having any one of the following structures:

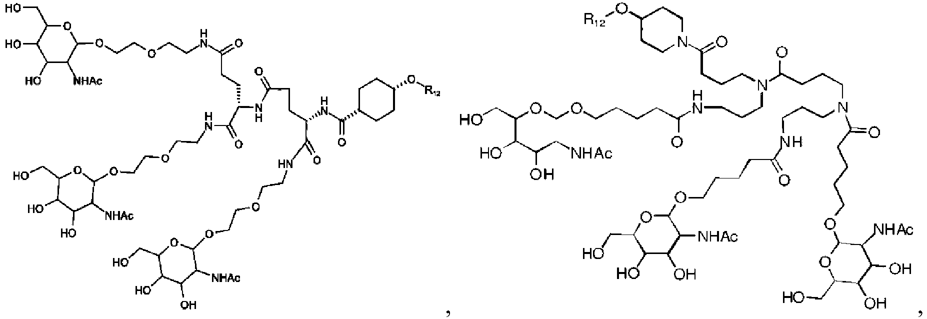

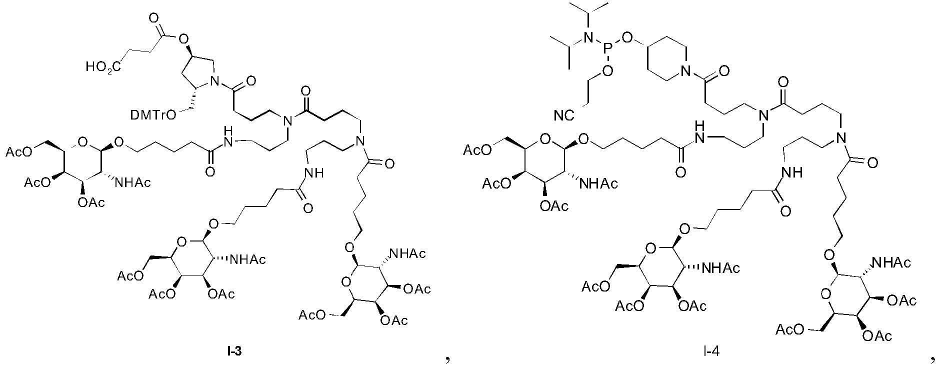

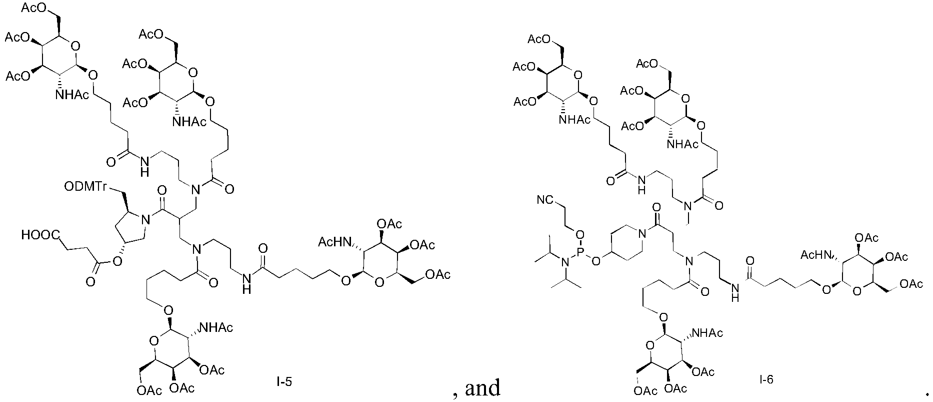

5. A compound, or a stereoisomer, tautomer or pharmaceutically acceptable salt thereof, having a structure represented by formula (II): 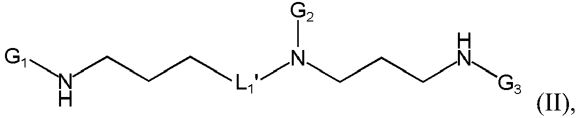 wherein:
L₁' is -N(R₁')-C(O)-(C₁₋₁₀ alkylene)- or -N(G₄)-L₂'-;
L₂' is C₁₆ alkylene substituted with R₁';
G₁, G₂, G₃, and G₄ are each independently 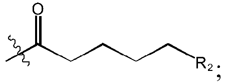
R₂ is a residue of a substituted or unsubstituted saccharide;
R₁' is 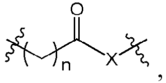 where n is any integer ranging from 0 to 10; and
X is absent or a linker, wherein the linker, if present, is -NH-(C₁₋₁₀ alkylene)-O-, -NH-(C₁₋₁₀ alkylene)-S-, -C₄₋₁₀ heterocycloalkylene-O-, -C₄₋₁₀ heterocycloalkylene-S-, -C₄₋₁₀ heterocycloalkylene-(C₁₋₆ alkylene)-O- optionally substituted with one or more Rₐ groups, or - C₄₋₁₀ heterocycloalkylene-(C₁₋₆ alkylene)-S- optionally substituted with one or more Rₐ groups, wherein the one or more Rₐ groups is each independently selected from -OH and -C₁₋₆ alkylene; and preferably, X is a linker.

6. The compound or the stereoisomer, tautomer or pharmaceutically acceptable salt thereof according to claim 5, satisfying at least one of the following conditions:
1) L₂' is C₂₋₄ alkylene substituted with R₁';
2) L₁' is -N(R₁')-C(O)-(C₁₋₆ alkylene)- or -N(G₄)-CH₂-CH(R₁')-CH₂-;
3) the linker, if present, is -NH-(C₄₋₈ alkylene)-O-, -C₄₋₁₀ heterocycloalkylene-O-, or -C₄₋₁₀ heterocycloalkylene-CH₂-O- optionally substituted with -OH; preferably, the linker, if present, is 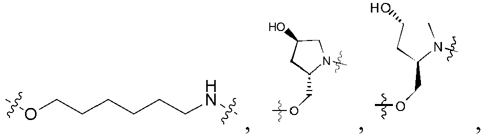 or 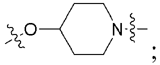
4) R₁' is selected from 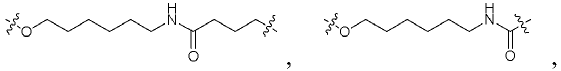 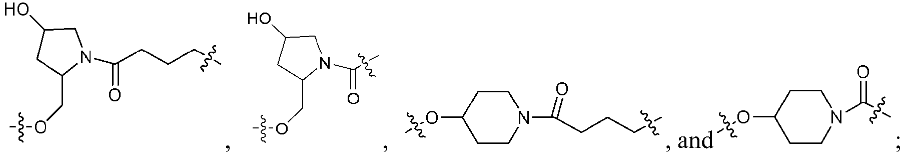 preferably, R₁' is selected from 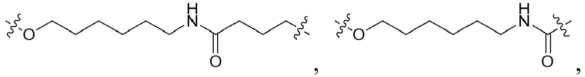 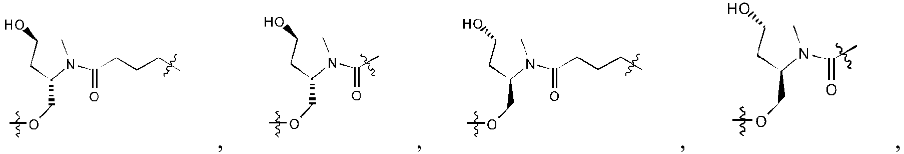 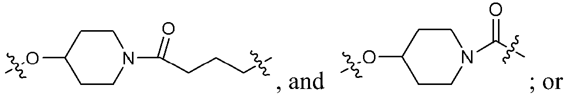
5) R₂ in G₁, G₂, G₃, and G₄ is each independently a residue of a compound selected from N-acetylgalactosamine, N-trifluoroacetylgalactosamine, N-formylgalactosamine, N-propionylgalactosamine, N-n-butyrylgalactosamine, and N-isobutyrylgalactosamine;
preferably, G₁, G₂, G₃, and G₄ are each independently

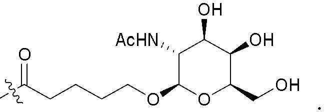

7. The compound or the stereoisomer, tautomer or pharmaceutically acceptable salt thereof according to claim 5, wherein the compound has a structure represented by formula (IIa) or formula (IIb): 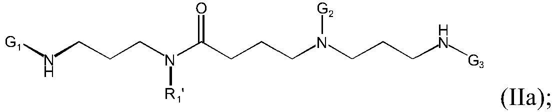 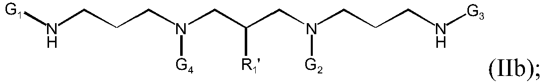 wherein:
in formula (IIa), R₁' is 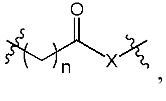 where n is any integer ranging from 1 to 6; and
in formula (IIb), R₁' is 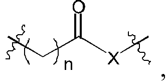 where n is 0.

8. The compound or the stereoisomer, tautomer or pharmaceutically acceptable salt thereof according to claim 5, wherein the compound has any one of the following structures:

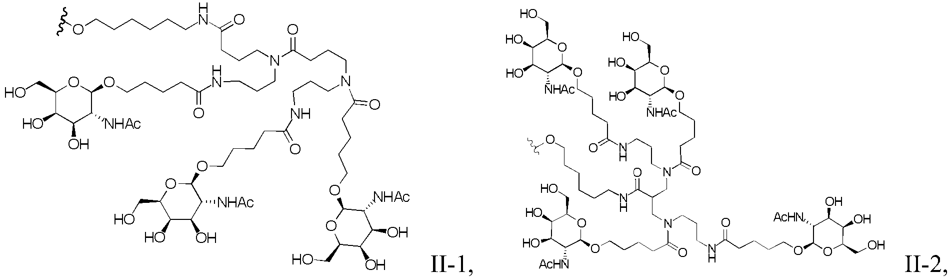

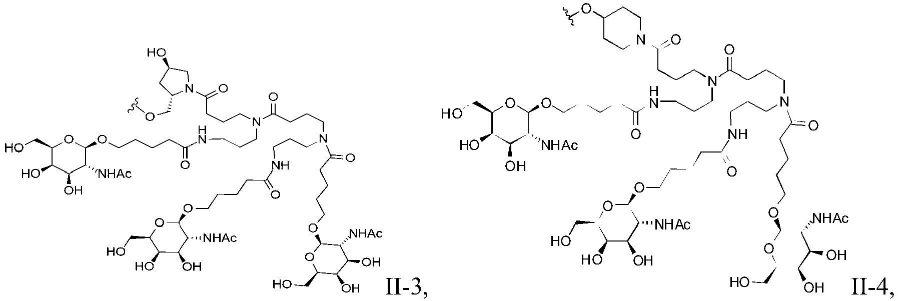

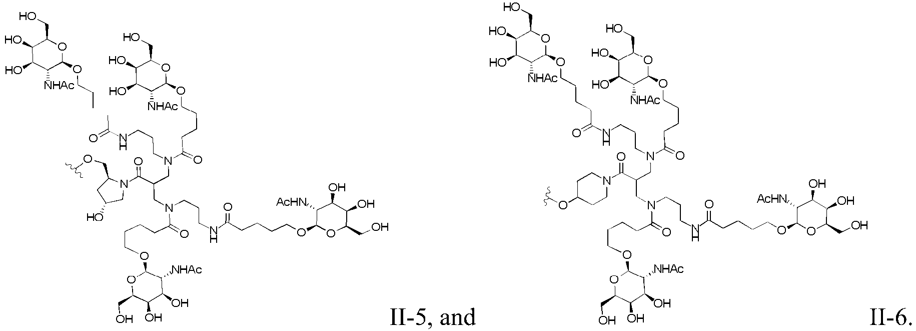

9. Use of the compound or the stereoisomer, tautomer or pharmaceutically acceptable salt thereof according to any one of claims 1 to 8 in the manufacture of a medicament, wherein:
preferably, the medicament is for use in the targeted delivery of an active pharmaceutical agent to cells;
preferably, the medicament is for use in the targeted delivery of an oligonucleotide to cells;
preferably, the cells are liver cells and/or renal cells; and
preferably, the medicament for use in the reduction of expression or activity of a target gene.

10. A conjugate, comprising:
an active pharmaceutical agent; and
a ligand conjugated to the active pharmaceutical agent, wherein the ligand is the compound or the stereoisomer, tautomer or pharmaceutically acceptable salt thereof according to any one of claims 5 to 8.

11. The conjugate according to claim 10, wherein:
the active pharmaceutical agent is an oligonucleotide; and
the conjugate comprises an oligonucleotide, and a ligand conjugated to the oligonucleotide, wherein the ligand is the compound or the stereoisomer, tautomer or pharmaceutically acceptable salt thereof according to any one of claims 5 to 8.

12. The conjugate according to claim 11, satisfying at least one of the following conditions:
i) the oligonucleotide is ASO, siRNA, or miRNA;
ii) the ligand is linked to the sense strand and/or the antisense strand in siRNA;
iii) the ligand is linked to the sense strand and/or the antisense strand via a phosphorothioate bond or a phosphate bond;
iv) the ligand is linked to the 5' end or 3' end of the sense strand;
v) the ligand is linked to the 5' end or 3' end of the antisense strand; or
vi) the ligand is linked to the oligonucleotide via the R1' group in the ligand, or the ligand is linked to the oligonucleotide via the linker of the R1' group in the ligand.

13. The conjugate according to claim 10, being a compound represented by formula (III), or the stereoisomer, tautomer or pharmaceutically acceptable salt thereof: 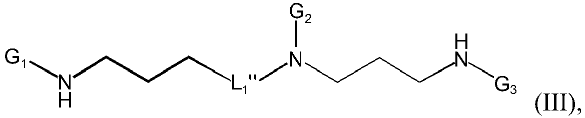 wherein:
L₁" is -N(R₁")-C(O)-(C₁₋₁₀ alkylene)- or -N(G₄)-L₂"-;
L₂" is C₁₋₆ alkylene substituted with R₁";
G₁, G₂, G₃, and G₄ are each independently 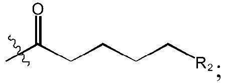
R₂ is a residue of a substituted or unsubstituted saccharide;
R₁" is -(C₀₋₁₀ alkylene)-C(O)-linker-R₁₂; and
R₁₂ is an active pharmaceutical agent, preferably an oligonucleotide.

14. The conjugate according to claim 13, satisfying at least one of the following conditions:
1) L₂" is C₂₋₄ alkylene substituted with R₁";
2) L₁" is -N(R₁")-C(O)-(C₁₋₆ alkylene)- or -N(G₄)-CH₂-CH(R₁ ")-CH₂-;
3) R₁₂ is ASO, siRNA, or miRNA;
4) G₁, G₂, G₃, and G₄ are each independently 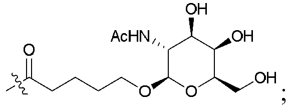
5) R₁" is selected from the group consisting of

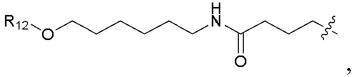

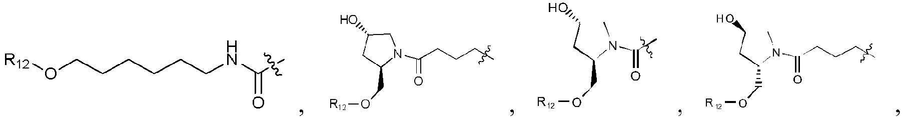

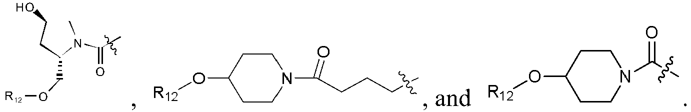

15. The conjugate according to claim 13, wherein the compound represented by formula (III) has a structure represented by formula (IIIa) or formula (IIIb):

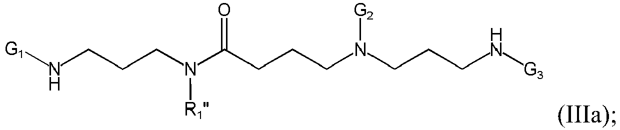

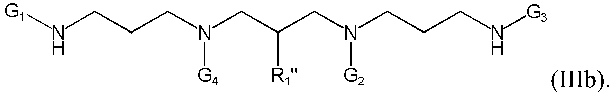

16. The conjugate according to claim 13, having any one of the following structures: 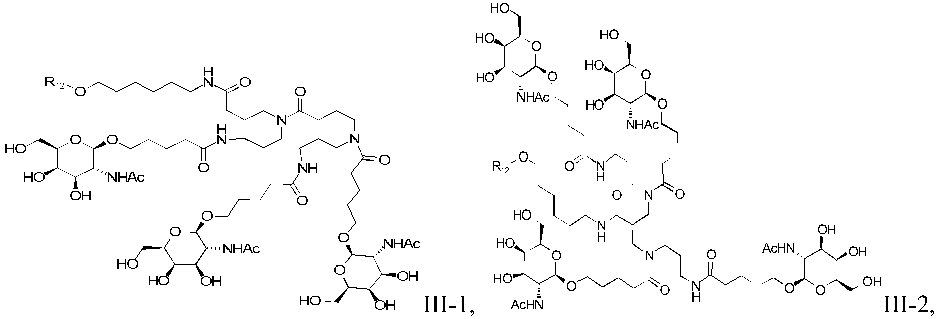 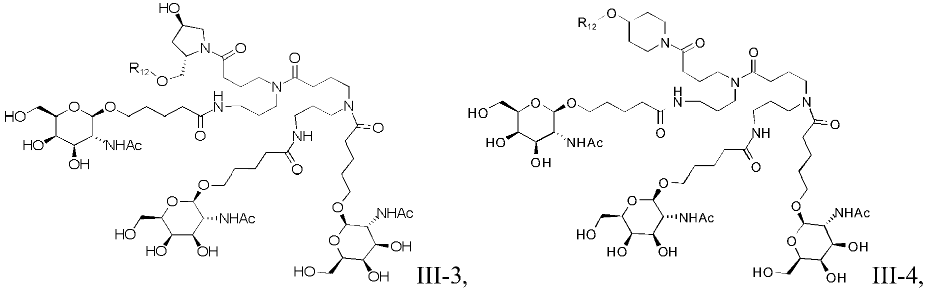 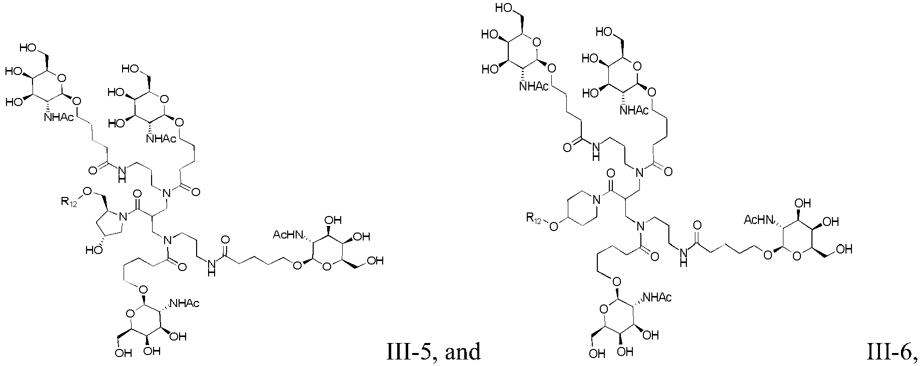 wherein:
R₁₂ is the active pharmaceutical agent, and preferably, an oligonucleotide; and
optionally, R₁₂ is ASO, siRNA, or miRNA.

17. The conjugate according to claim 13, having any one of the following structures: 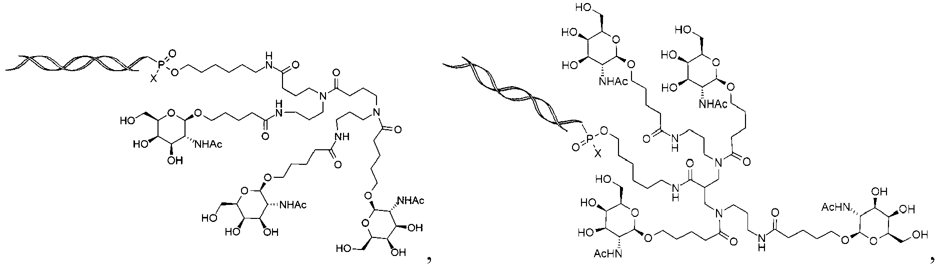 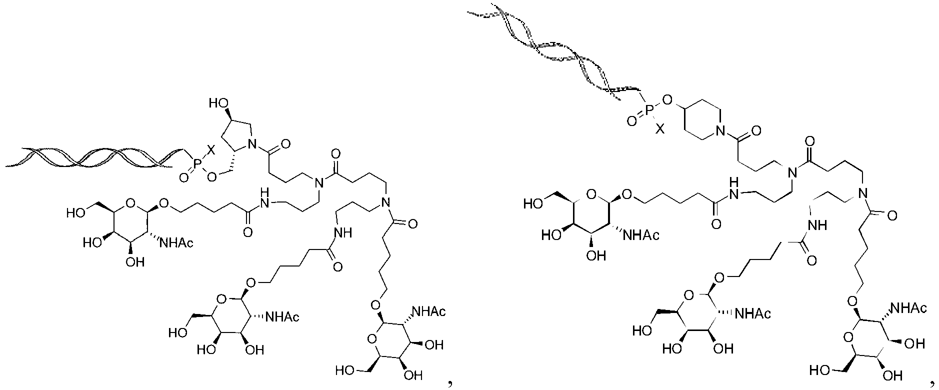 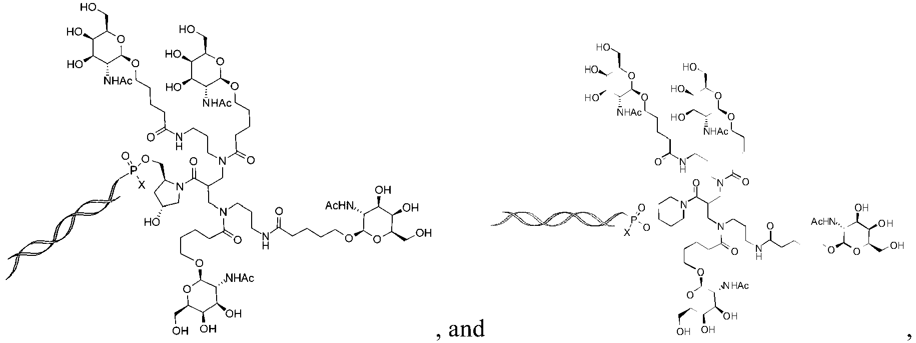 wherein: 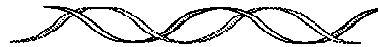
represents an oligonucleotide; and
X represents O or S.

18. A pharmaceutical composition, comprising:
the compound or the stereoisomer, tautomer or pharmaceutically acceptable salt thereof according to any one of claims 5 to 8, or the conjugate according to any one of claims 10 to 17; and
optionally, a pharmaceutically acceptable carrier.

19. Use of the conjugate according to any one of claims 10 to 17 or the pharmaceutical composition according to claim 18 in:
the manufacture of a medicament for preventing and/or treating a disease;
the manufacture of a medicament for reducing expression or activity of a target gene;
the prevention and/or treatment of a disease;
the reduction of expression or activity of a target gene; or
targeted delivery of an active pharmaceutical agent to cells, wherein the cells are preferably liver cells and/or renal cells.

20. The conjugate according to any one of claims 10 to 17 or the pharmaceutical composition according to claim 18 for use in the prevention and/or treatment of a disease, and/or for use in targeted delivery of an active pharmaceutical agent to cells, wherein the cells are preferably liver cells and/or renal cells.

21. A method for preventing and/or treating a disease, comprising:
administering to a subject a pharmaceutically acceptable amount of the conjugate according to any one of claims 10 to 17 or the pharmaceutical composition according to claim 18.
